(19) Europäisches Patentamt
European Patent Office
Office européen des brevets

(11) EP 2 605 769 B1

(12) # EUROPEAN PATENT SPECIFICATION

(45) Date of publication and mention of the grant of the patent:
**27.07.2016 Bulletin 2016/30**

(21) Application number: **11748898.1**

(22) Date of filing: **16.08.2011**

(51) Int Cl.:
***A61K 31/122*** *(2006.01)* ***C07C 50/28*** *(2006.01)*

(86) International application number:
**PCT/EP2011/004123**

(87) International publication number:
**WO 2012/022468 (23.02.2012 Gazette 2012/08)**

(54) **BENZOQUINONE DERIVATIVES FOR THE TREATMENT OF MITCHONDRIAL EYE DISEASES**

BENZOCHINONDERIVATE ZUR BEHANDLUNG MITOCHONDRIALER AUGENKRANKHEITEN

DÉRIVÉS DE BENZOQUINONE EN TANT QUE MÉDICAMENTS POUR LE TRAITEMENT DE MALADIES MITOCHONDRIALES DES YEUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**11.04.2011 US 201161473835 P**
**20.12.2010 US 201061424978 P**
**16.08.2010 US 373972 P**
**11.04.2011 EP 11003037**
**20.12.2010 EP 10015842**
**16.08.2010 EP 10008542**

(43) Date of publication of application:
**26.06.2013 Bulletin 2013/26**

(73) Proprietor: **Santhera Pharmaceuticals (Schweiz) AG**
**4410 Liestal (CH)**

(72) Inventors:
- **FEUERER, Achim**
  **79424 Auggen (DE)**
- **GUEVEN, Nuri**
  **79618 Rheinfelden (DE)**
- **HOFFMANN-ENGER, Barbara**
  **CH-4106 Therwil (CH)**
- **ERB, Michael**
  **CH-4416 Gelterkinden (CH)**
- **DEPPE, Holger**
  **CH-4414 Füllinsdorf (CH)**
- **DALLMANN, Robert**
  **CH-3013 Bern (CH)**
- **HAEFELI, Roman**
  **CH-4056 Basel (CH)**
- **HEITZ, Fabrice**
  **F-68870 Bartenheim (FR)**

(74) Representative: **Grünecker Patent- und Rechtsanwälte**
**PartG mbB**
**Leopoldstraße 4**
**80802 München (DE)**

(56) References cited:
**EP-A1- 0 788 793** **EP-A2- 0 201 196**
**WO-A2-98/02149**

- **KAYOKO OKAMOTO ET AL: "Synthesis, Metabolism, and in Vitro Biological Activities of 6-(10-Hydroxydecyl)-2, 3-dimethoxy-5-methyl-1, 4-benzoquinone (CV-2619)-Related Compounds", CHEMICAL AND PHARMACEUTICAL BULLETIN, PHARMACEUTICAL SOCIETY OF JAPAN, TOKYO, JP, vol. 36, no. 1, 1 January 1988 (1988-01-01), pages 178-189, XP009143726, ISSN: 0009-2363**
- **DUVEAU D Y ET AL: "Synthesis and characterization of mitoQ and idebenone analogues as mediators of oxygen consumption in mitochondria", BIOORGANIC & MEDICINAL CHEMISTRY, PERGAMON, GB, vol. 18, no. 17, 4 August 2010 (2010-08-04), pages 6429-6441, XP027218831, ISSN: 0968-0896 [retrieved on 2010-08-04]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 605 769 B1

- **DATABASE CA [Online] CHEMICAL ABSTRACTS SERVICE, COLUMBUS, OHIO, US; "Stable 6-(10-hydroxydodecyl)-2,3-dimethoxy-5-methyl-1,4-benzoquinone microcrystals", XP002668984, retrieved from STN Database accession no. 1983:476854 & DATABASE WPI Week 198325 Thomson Scientific, London, GB; AN 1983-59523K & JP 58 077839 A (TAKEDA CHEM IND LTD) 11 May 1983 (1983-05-11)**
- **Daniel Jarovsky ET AL: "Mitochondrial Diseases: A review", Reviewing Basic Sciences, 1 January 2006 (2006-01-01), pages 343-350, XP055018708, Retrieved from the Internet: URL:http://apps.einstein.br/REVISTA/arquivos/PDF/Einstein_vol4_n4_2006_p343.pdf [retrieved on 2012-02-08]**

Remarks:

The file contains technical information submitted after the application was filed and not included in this specification

## Description

### Field of the Invention

The present invention relates to benzoquinone derivatives which are efficient as modulators of mitochondrial function and as such are useful for the treatment of pathological conditions where mitochondrial function is impaired.

### Background of the Invention

Mitochondria, sometimes described as "cellular power plants" because they generate most of the cell's supply of adenosine triphosphate (ATP), are essential to eukaryotic life. In addition to supplying cellular energy, mitochondria are also involved in a range of other processes, such as signalling, cellular differentiation, cell death, as well as the control of the cell cycle and cell growth. Mitochondria have been implicated in several human diseases, including mitochondrial disorders and cardiac dysfunction and may play a role in the aging process.

With their central place in cell metabolism, mitochondrial dysfunction is an important factor in a wide range of human diseases.

Chrysostomou et al. (Chrysostomou V, Trounce IA, Crowston JG. Ophthalmic Res. 2010;44(3): 173-8) suggested that mitochondrial dysfunction, inherited or as a cause or consequence of injury, renders ocular cells (in particular retinal ganglion cells) sensitive to degeneration. Therapeutic approaches that target mitochondria should therefore provide a general means of protecting lens and retinal ganglion cells from degeneration, regardless of the etiology of the disease.

Hence, compounds able to protect or recover mitochondrial function in the eye should be useful as therapeutics for the treatment of diseases associated with impaired mitochondrial function in the eye. Such diseases may on the one hand be genetic mitochondrial disorders of the eye, i.e. ophthalmological indications such as Leber's hereditary optic neuropathy (LHON), autosomal dominant optic atrophy (DOA), or ophtalmological indications displaying mitochondrial dysfunction such as macular degeneration, glaucoma, retinopathy, cataract, optic disc drusen (ODD). Furthermore, some genetic, mitochondrial-neurodegenerative disorders that might not be ophthalmological diseases *per se,* such as MELAS (mitochondrial myopathy, encephalomyopathy, lactic acidosis, stroke-like symptoms), MERFF (myoclonic epilepsy with ragged red fibers), MNGIE (myoneurogenic gastrointestinal encephalomyopathy), Kearns-Sayre syndrome, CoQ10 deficiency, or mitochondrial complex deficiencies (i.e. complex I, II, III, IV, V deficiency, and CPEO) can well exhibit an ophthalmological component among their various symptoms and are thus falling as well under the scope of the above mentioned diseases associated with impaired mitochondrial funtion.

Moreover, mitochondrial disorders often present as neurological disorders, but also manifest as multi-system disorders, including myopathy, diabetes, multiple endocrinopathy, or a variety of other systemic manifestations. These disorders can be caused by mutations of mitochondrial DNA, by mutations of nuclear genes directly coding for oxidative phosphorylation enzymes and by defects in nuclear genes that are generally important for mitochondrial function. Environmental influences may also interact with hereditary predispositions and cause mitochondrial disease. For example, there is a strong link between pesticide exposure and the later onset of Parkinson's disease. Numerous other pathologies involving mitochondrial dysfunction include schizophrenia, bipolar disorder epilepsy, stroke and autism (Jou et al. Chang Gung Med J. 2009; 32(4):370-9), dementia, Alzheimer's, Parkinson's and Huntington's disease (Yang et al. DNA Repair (Amst). 2008; 7(7):1110-20), cardiovascular disease (Puddu et al. J Biomed Sci. 2009; 16:112), malignancies (Singh & Kulawiec; Methods Mol Biol. 2009; 471:291-303), retinitis pigmentosa, metabolic syndrome, anorexia, obesity and diabetes mellitus (Burchell et al. Expert Opin Ther Targets 2010; 14(4):369-85; Pieczenik & Neustadt Exp Mol Pathol. 2007; 83(1):84-92).

Multiple sclerosis is the most common non-traumatic neurological disease in young adults, with a prevalence of 1:1000 in Northern Europe and North-America (Compston A Int MS J .2003. 10:29-31). At first, the disease course is generally episodic; exacerbations are followed by periods of remission which are characterized by focal infiltration of leukocytes and demyelination in the white matter. Gradually the disease becomes more progressive, where demyelination of the grey matter and axonal degeneration in the white matter become more prominent (Hafler DA J. Clin. Invest. 2004; 113:788-794). Available therapies are mainly immunomodulatory, which are effective in reducing the number of relapses. Disease progression, however, remains unchanged by these therapies (Filippini G et al. Lancet 2003; 361:545-552), demonstrating the need for novel therapeutic strategies oriented towards neuroprotection.

It is thought that one of the critical underlying defects of these disorders is the deficiency of damaged mitochondria to provide sufficient energy to allow normal cellular functions and/or their characteristic to generate excess oxidative stress. As a consequence of this energy deprivation and macromolecular damage, cells die. This in turn impairs tissue function and causes disease pathology. This connection is most obvious in mitochondrial disorders that are characterized by pathologies in tissues with high energy demand such as brain and muscle cells, where cell loss or impaired cellular function in these tissues is intimately linked to the phenotype of the patient.

Although there are multiple therapeutic approaches for the indications listed above, however, only very few of those are addressing the underlying mitochondrial dysfunction. Thus, there still exists a need for novel therapeutic approaches for treating disorders caused by impaired mitochondria.

WO-A-2006130775 describes an attempt to treat or suppress mitochondrial diseases by modulating energy biomarkers such as lactic acid levels, levels of NAD, NADP, NADH and NADPH, and cytochrome C parameters. The compounds disclosed in the application have been tested for their ability to rescue human dermal fibroblasts from FRDA patients from oxidative stress. However, the data are not represented in the application.

EP-A-0201196 *relates to benzoquinone derivatives which may be used in the prophylaxis and treatment of ischemic diseases such as cerebral apoplexy or allergic diseases based on their postulated inhibitory activity of the generation and release of SRS-A.*

K. Okamoto et al. discuss the synthesis, metabolism and in vitro biological activities of 6-(10-hydroxydecyl)-2,3-dimethoxy-5-methyl-1,4-benzoquinone-related compounds in Chem. Pharm. Bull. 36(1), 178-189 (1988*).*

D. Y. Duveau et al. prepared and investigated analogues of mitoQ and idebenone to define the structural elements that support oxygen consumption in the mitochondrial respiratory chain in Bioorg. Med. Chem. 18 (2010), 6429-6411*.*

JP-A-58077839 *discloses 6-(10-hydroxydodecyl)-2,3-dimethoxy-5-methyl-1,4-benzoquinone as a therapeutic agent for malignant hypertension and cerebrovascular disorder.*

It is the object of the present invention to provide a novel therapeutic treatment for pathological conditions where mitochondrial function is impaired.

Surprisingly, it has been found that benzoquinone derivatives according to formula (I) shown below provide a solution to the object of the present invention.

**Summary of the Invention**

The present invention relates to benzoquinone derivatives according to formula (I)

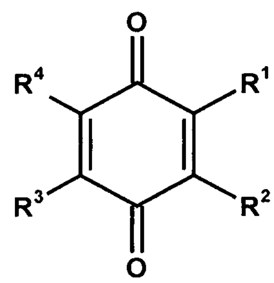

(I)

wherein $R^1$, $R^2$, $R^3$ and $R^4$ are defined as below for use in the treatment of disorders wherein mitochondrial function is impaired.

The benzoquinone derivatives of formula (I) are particularly useful in the treatment of mitochondrial diseases.

The mitochondrial diseases are selected from Leber's hereditary optic neuropathy (LHON), autosomal dominant optic atrophy (DOA), macular degeneration, glaucoma, retinopathy, cataract, and optic disc drusen (ODD).

The present invention further relates to a method for treating disorders wherein mitochondrial function impaired, the method comprising administering an effective amount of a compound according to formula (I) to a subject in need thereof.

In a preferred embodiment, the subject is a mammal, more preferably a human.

**Brief description of the drawings**

Figure 1 shows the protective effect of compound IA in RGC-5 cells as *in vitro* model of mitochondrial dysfunction in ophtalmological cells, 1 day after rotenone challenge.

Figure 2 shows the comparison of compound IA and propentophylline on cellular survival of RGC cells in response to mitochondrial impairment by rotenone. While cellular protection by propentophylline was not significant (p>0.05), the effect of compound IA was highly significant (p=0.0005).

Figure 3 shows the effect of compound IA pre-treatment on visual acuity in an in vivo mouse model for eye disorders characterized by mitochondrial dysfunction.

**Detailed Description of the Invention**

The present invention relates to a compound represented by the following formula

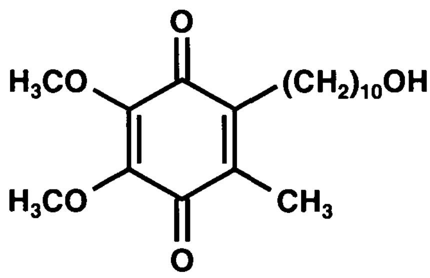

and tautomers, solvates or pharmaceutically acceptable salts thereof,
for use in the oral treatment of a mitochondrial disease selected from the group consisting of Leber's hereditary optic neuropathy (LHON), autosomal dominant optic atrophy (DOA), macular degeneration, glaucoma, retinopathy, cataract, and optic disc drusen (ODD).

In a preferred embodiment in combination with any of the above or below embodiments, the compound is represented by the following formula

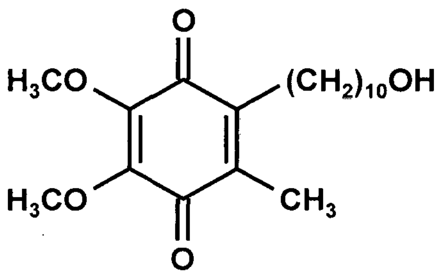

,

i.e. the compound is idebenone.

In a preferred embodiment in combination with any of the above or below embodiments, the mitochondrial disease is selected from the group consisting of Leber's hereditary optic neuropathy (LHON), autosomal dominant optic atrophy (DOA), macular degeneration, and glaucoma.

In a preferred embodiment in combination with any of the above or below embodiments, the mitochondrial disease is Leber's hereditary optic neuropathy (LHON).

In a preferred embodiment in combination with any of the above or below embodiments, the mitochondrial disease is autosomal dominant optic atrophy (DOA).

In a preferred embodiment in combination with any of the above or below embodiments, the mitochondrial disease is macular degeneration.

In a preferred embodiment in combination with any of the above or below embodiments, the mitochondrial disease is glaucoma.

In a preferred embodiment in combination with any of the above or below embodiments, the mitochondrial disease is retinopathy.

In a preferred embodiment in combination with any of the above or below embodiments, the mitochondrial disease is cataract. In other words, the disease is cataract formation.

In a preferred embodiment in combination with any of the above or below embodiments, the mitochondrial disease is optic disc drusen (ODD).

In another embodiment, the present invention relates to benzoquinone derivatives according to formula (I)

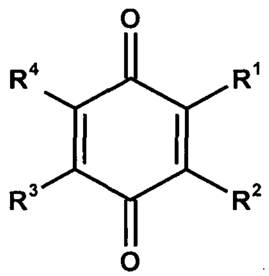

(I)

and the enantiomers, tautomers, solvates or pharmaceutically acceptable salts thereof wherein

a)

$R^1$ is a substituent represented by formula (II):

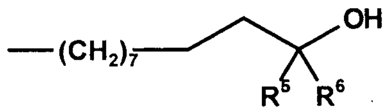

(II)

wherein

$R^5$ and $R^6$ are both hydrogen atoms, or
$R^5$ is a hydrogen atom and $R^6$ is an ethyl group, or
$R^5$ and $R^6$ are both methyl groups,

$R^2$ is a methyl group,
$R^3$ is a methoxy group, and
$R^4$ is a methoxy group;

for use in the treatment of disorders wherein mitochondrial function is impaired, in particular in the treatment of mitochondrial disorders.

In a preferred embodiment in combination with any of the above or below embodiments, the mitochondrial disease is selected from the group consisting of Leber's hereditary optic neuropathy (LHON), autosomal dominant optic atrophy (DOA), macular degeneration, glaucoma, retinopathy, cataract, optic disc drusen (ODD).

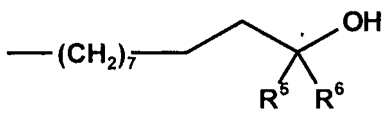

In a preferred embodiment in combination with any of the above or below embodiments, the compound is a compound of formula (I), wherein

$R^1$ is a substituent represented by formula (II):

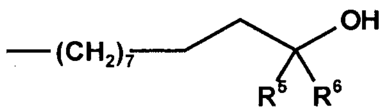

(II)

wherein

$R^5$ and $R^6$ are both hydrogen atoms,

$R^2$ is a methyl group,
$R^3$ is a methoxy group, and
$R^4$ is a methoxy group.

In a preferred embodiment in combination with any of the above or below embodiments, the compound is a compound of formula (I), wherein

$R^1$ in formula (I) is a substituent represented by formula (II):

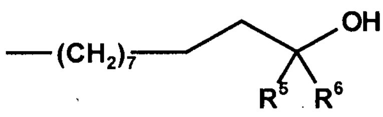

(II)

wherein

$R^5$ is a hydrogen atom and $R^6$ is an ethyl group,

$R^2$ is a methyl group,
$R^3$ is a methoxy group, and
$R^4$ is a methoxy group.

In a preferred embodiment in combination with any of the above or below embodiments, the compound is a compound of formula (I), wherein

$R^1$ in formula (I) is a substituent represented by formula (II):

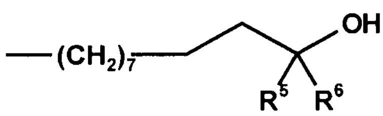

(II)

wherein

$R^5$ and $R^6$ are both methyl groups,

$R^2$ is a methyl group,
$R^3$ is a methoxy group, and
$R^4$ is a methoxy group.

In another embodiment, the present invention is directed to the use of a compound represented by the following formula

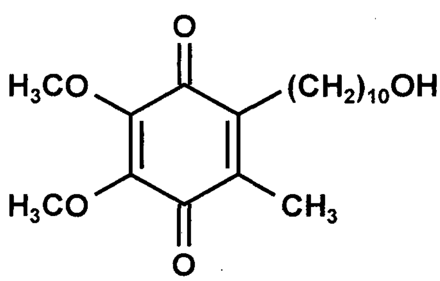

and tautomers, solvates or pharmaceutically acceptable salts thereof,
for the preparation of a medicament for the oral treatment of a mitochondrial disease selected from the group consisting of Leber's hereditary optic neuropathy (LHON), autosomal dominant optic atrophy (DOA), macular degeneration, glaucoma, retinopathy, cataract, and optic disc drusen (ODD).

In another embodiment, the present invention is directed to the use of a compound according to general formula (I)

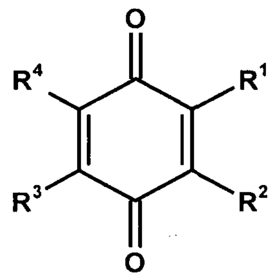

wherein $R^1$ to $R^4$ are defined as above for the preparation of a medicament in the treatment of mitochondrial diseases.

In a preferred embodiment in combination with any of the embodiments above and below, compounds according to formula (I) are used wherein $R^1$ represents formula (II) and $R^2$ to $R^6$ are defined as above. More preferably, $R^1$ represent formula (II) and $R^5$ and $R^6$ both represent hydrogen atoms.

According to the invention, the compounds of formula (I) are for use in the treatment of mitochondrial disorders.

The compounds of formula (I) *and similar compounds* are known compounds and have been previously described.

The compound of formula (I) wherein $R^1$ represents formula (II) with $R^5$ and $R^6$ both representing hydrogen atoms is known as Idebenone (in the following referred to as Compound IA). This compound is already used as a nootropic drug and has also been described to stimulate nerve growth factor, a characteristic that could be important in the treatment of Alzheimer's and other neurodegenerative diseases. WO 2006100017 describes the use of Compound IA in the treatment of muscular dystrophies such as Duchenne muscular dystrophy and Becker muscular dystrophy. Compound IA and its preparation are first described in the specification of Japanese Patent Examined Publication No. 3134/1987 filed by Takeda Chemical Industries, Ltd.

The compound of formula (I) wherein $R^1$ represents formula (II) with $R^5$ being a hydrogen atom and $R^6$ being an ethyl group (in the following referred to as Compound IB) is described in JP 58077839. It is suggested to use the drug in the treatment of malignant hypertension and cerebrovascular disorders.

The compound of formula (I) wherein $R^1$ represents formula (II) with $R^5$ and $R^6$ both representing a methyl group (in the following referred to as Compound IC) and its synthesis have been only described in Okamoto et al., Chem. Pharm. Bull. 1988, 36, 178 and EP-A-0201196. The authors discussed various possible therapeutic uses of the compounds described in EP-A-0201196, e.g. the therapy of ischemic diseases such as cerebral apoplexy and cardiac insufficiency. They also mentioned an inhibitory action of the compounds described therein on the generation and release of SRS-A in mammals as well as their circulatory disturbance-improving activity. It should, however, be noted that the proposed treatments have not been substantiated with pharmacological data. To date, to the best of our knowledge a practical therapeutic use of Compound IC is not known.

The compound of formula (I) wherein $R^1$ is a $-(CH_2)_{10}-OH$ group and each of $R^2$ to $R^4$ represents a methyl group can be considered as an analogue to Idebenone (in the following referred to as Compound ID). A general method for the synthesis of benzoquinone compounds such as Compound ID is described in EP-A-0038674. Said patent application also investigates the role of benzoquinone compounds such as Compound ID in the inhibition of the generation and release of SRS-A and contemplates their potential use in the treatment of allergic diseases.

The compound of formula (I) wherein $R^1$ is a $-(CH_2)_{10}-CH_3$ group, both of $R^2$ and $R^4$ represent a hydroxy group and $R^3$ is a hydrogen atom (in the following referred to as Compound IE) is also known under the name Embelin as it has been identified primarily from the *Embelia ribes* plant but is nowadays commercially available through various suppliers. Said compound has been shown to exhibit anti-tumor, anti-inflammatory, and apoptotic activities through an unknown mechanism.

For example, it is stated in Molecular Carcinogenesis (2010) 49(4), 324-336 that Embelin, the small molecular inhibitor of XIAP, possesses a wide spectrum of biological activities with strong inhibition of nuclear factor kappa B and downstream antiapoptotic genes but that the mechanism of its cell death induction is not known. Further studies suggest that Embelin can act as a competitive antioxidant in physiological conditions (Chemico-Biological Interactions (2007), 167(2), 125-134).

The compound of formula (I) wherein $R^1$ is a $-(CH_2)_{10}-CH_3$ group, both of $R^2$ and $R^4$ represent a methoxy group

and $R^3$ is a hydrogen atom is a derivative to Embelin (in the following referred to as Compound IF). A method for the synthesis of Compound IF is, for example, given in Tetrahedron (1998), 54(49), 14791-802. A therapeutic use has not been indicated for Compound IF, however, the compound may possibly show some activity in hepatitis C therapy as can be deduced from a study about the inhibitory effects of plant extracts commonly used in Sudanese traditional medicine on hepatitis C virus protease (Phytotherapy Research (2000), 14(7), 510-6). It was found that two benzoquinone compounds extracted from Embelia schimperi, namely embelin and 5-O-methylembelin, are potent hepatitis C virus protease inhibitors.

The compounds according to formula (I) are modulators of mitochondrial disorders and are thus useful in the treatment of mitochondrial diseases in general.

The oral biovailability of compounds of formula (I) is generally very low as exemplified for compound IA (Torii H, Yoshida K, Kobayashi T, Tsukamoto T, Tanayama S. J Pharmacobiodyn. 1985 Jun;8(6):457-67).Though they are readily and fast absorbed in the gut when administered orally, the major part of the drug is metabolized by an extremely strong first-pass effect in the liver, and hence, only smaller portions of the dose is circulating in the blood stream.

Attempts to bypass the strong first-pass effect of idebenone (compound IA) include to administer the compound via routes other than oral. Parenteral formulations are difficult to obtain as idebenone exhibits only very low water solubility. US2010/0130619, US2010/0129431A1, US2010/0215725A1 and US2010/0099775A1 describe a parenteral formulation of idebenone suitable for intravenous injection or infusion.

WO2008/019769 discloses a transmucosal formulation of idebenone to circumvent the strong first-pass effect. Even though this formulation is taken via the mouth, the processing of the drug by the body is completely different compared to the oral administration. In contrast to an oral administration, the transmucosal (e.g. sublingual) formulation of a drug leads to absorption via the mucosa which enables the drug to reach the blood stream even without being processed by the gastro-intestinal tract, and hence, avoids the first-pass effect in the liver. The applicants state that the drug dose can be significantly lowered compared to the oral dose while reaching similarly high plasma levels and claim such formulation to be useful in the treatment of mitochondrial, neurological and neuromuscular diseases, including FRDA (Friedreich's Ataxia), LHON (Leber's Hereditary Optic Neuropathy), MELAS (mitochondrial myopathy, encephalopathy, lactic acidosis with stroke-like episodes) and mitochondrial myopathies.

The consequence of avoiding the first-pass effect is that higher concentrations of the drug are circulating in the blood stream and finally that higher concentrations of the drug can be achieved in the tissue relevant for a given disease.

A parenteral, e.g. intraveneous administration, of a compound is often considered either not practicable or causing significant nursing cost in the setting of a genetic (and consequently chronic) disease, where patients are home-based and not in a hospital but have to take a drug life-long.

A transmucosal formulation, might - in particular in the case of compounds of formula (I) - due to their intense colour and potential bad taste also be hampered by a reduced patient compliance.

Another way to bypass the strong first-pass effect is to administer the compounds of formula (I) by topical formulation, including dermal, buccal, sublingual and intraocular formulation.

Beyond the high first-pass effect, a further drawback of compounds of formula (I), and again exemplified with compound IA as a representative for this compound class, is the fact, that these compounds are reaching the brain only in very limited amounts when given orally. For example, it is known from the literature (Torii H, Yoshida K, Kobayashi T, Tsukamoto T, Tanayama S. J Pharmacobiodyn. 1985 Jun;8(6):457-67) that an oral dose of 10 mg/kg of idebenone (compound IA) in rats leads to plasma levels of approximately 1000 ng/ml of total idebenone as measured by radioactivity after oral dosing of radioactive labeled compound IA. The total concentration means idebenone including its metabolites. Taken the correction factor calculated from Torii et al to obtain the levels of the unmetabolized parent compound IA, this results in idebenone plasma levels of 3 ng/ml. The corresponding levels in the brain are 10 ng/ml of total idebenone (which again means including the metabolites), resulting in only 0.03 ng/ml for parent idebenone itself. As a result, the ratio of brain vs plasma levels is about 1%. This is in line with the findings of Nagai et al (Nagai Y, Yoshida K, Narumi S, Tanayama S, Nagaoka A. Brain distribution of idebenone and its effect on local cerebral glucose utilization in rats. Arch Gerontol Geriatr. 1989;8(3):257-72), where higher oral doses of idebenone (100 mg/kg) are leading to similar low brain- to-plasma ratio of 1-2% in rats.

Drugs that need to reach the brain to exert their action, have to cross the so-called blood brain barrier (BBB). Efflux pumps like P-glycoproteins (P-gp) and multidrug resistance proteins (MRP) are causing the major hurdle for substances to cross the BBB and are protecting the brain against penetration of potentially toxic substances.

The eye is usually regarded as part of the central nervous system. This becomes evident in the fact, that, very similar to the blood-brain-barrier, the blood-retinal barrier keeps the retina protected against unwanted diffusion of potentially toxic compounds. Indeed, the blood brain barrier and the blood-retinal barrier are together called the blood-neural barrier (Invest Ophthalmol Vis Sci. 2005 Mar, 46(3), 1047-53: Functional characterization and comparison of the outer blood-retina barrier and the blood brain barrier: Steuer H et al. and Kim, JH et al.).

Thus, knowing of the very low levels of compound IA reaching the brain after oral administration, one would expect that compounds of this type, in particular compounds of formula (I), consequently will also not reach the eye in

significant, i.e. therapeutically relevant levels when given orally.

Hence, it was highly surprising, that, after oral administration of clinically relevant doses of Compound IA, concentrations of Compound IA in the eye fluid of mice (in aqueous and vitreous humor) could be detected that were several times higher than those previously measured in the brain of rodents and dogs (Torii H, Yoshida K, Kobayashi T, Tsukamoto T, Tanayama S. J Pharmacobiodyn. 1985 Jun;8(6):457-67).

In the present invention, pharmacokinetical data are disclosed demonstrating significant levels of compound IA in vitreous and aqueous humor of the eye after single oral administration.

It has thus been concluded for the first time that oral formulations of compounds of formula (I), in particular Compound IA, could be used to efficiently counteract diseases of the eye that are associated with mitochondrial impairment.

It would be highly desirable that compounds of formula (I) could be administered in a simple oral formulation which is perfectly fitting with patient compliance in home-based patients with a need of life-long treatment.

The discovery of surprisingly high levels of compounds of formula (I), as exemplified with oral administration of idebenone (compound IA) in the eye, and hence, the present invention will build a strong rationale for clinicians and regulatory authorities to investigate in studies and finally approval of drugs like oral idebenone for the use in mitochondrial eye diseases as set forth herein.

The eye levels of compound IA, obtained after oral administration, are sufficient to cause a significant recovery of visual loss in an animal model (Zhang X., Jones D., Gonzalez-Lima F. Mouse model of optic neuropathy caused by mitochondrial complex I dysfunction. Neurosci. Lett., 2002.) that is relevant for diseases with underlying mitochondrial impairment in the retina.

On the one hand, these diseases are ophthalmological mitochondrial diseases such as Leber's hereditary optic neuropathy (LHON), autosomal dominant optic atrophy (DOA), and ophtalmological disorders displaying mitochondrial dysfunction such as macular degeneration, glaucoma, retinopathy, cataract, optic disc drusen (ODD).

On the other hand, these diseases are genetic neurodegenerative mitochondrial diseases with an ophthalmological component among the various symptoms such as MELAS (mitochondrial myopathy, encephalomyopathy, lactic acidosis, stroke-like symptoms), MERFF (myoclonic epilepsy with ragged red fibers), MNGIE (myoneurogenic gastrointestinal encephalomyopathy), Kearns-Sayre syndrome, CoQ10 deficiency, or mitochondrial complex deficiencies (i.e. complex I, II, III, IV, V deficiency, and CPEO).

As used herein, the following diseases are designated as a "mitochondrial disease": Leber's hereditary optic neuropathy (LHON), autosomal dominant optic atrophy (DOA), macular degeneration, glaucoma, retinopathy, cataract, optic disc drusen (ODD), mitochondrial myopathy, encephalomyopathy, lactic acidosis, stroke-like symptoms (MELAS), myoclonic epilepsy with ragged red fibers (MERRF), myoneurogenic gastrointestinal encephalomyopathy (MNGIE), Kearns-Sayre syndrome, CoQ10 deficiency, and mitochondrial complex deficiencies (1-5, CPEO).

Idebenone (compound IA) is known to be a stimulator of neuronal growth factor (NGF) (Takeuchi, R., Murase, K., Furukawa, Y., Furukawa, S., Hayashi, K. Stimulation of nerve growth factor synthesis/secretion by 1,4-benzoquinone and its derivatives in cultured mouse astroglial cells. FEBS Lett. 1990, 261, 63-66).

Propentophylline is a compound from a completely different compound class and has also been described as stimulator of NGF (Shinoda et al, Stimulation of nerve growth factor synthesis/secretion by propentophylline in cultured mouse astroglial cells, Biochem. Pharmacol., vol. 39, 1990, 1813-1816). In WO2000/032197, data are disclosed that show positive effects of propentophylline in a retinal ganglion cells (RGC) survival assay. Solely from the data obtained from propentophylline, the applicants conclude that other compounds that are also known to be NGF stimulators - though from completely different chemical compound classes - have similar benefical effects on the survival of RGC. Consequently, the applicants claim idebenone and some other compounds from various chemical compound classes to be useful in the treatment of retina or optic nerve head neuropathy.

Moreover, the applicants state their compounds may be used orally. This is in accordance with the mechanism of action which - if really building the rational between compounds and disease treatment - does not require the compound itself to build levels in the eye but rather elsewhere in the body to stimluate the production of NGF which then in turn reaches the relevant tissues, e.g. the eye.

With regards to pathological mitochondrial ophthalmological conditions, the relevance of the RGC survival assay used in WO2000/032197 to demonstrate positive effects of propentophylline is highly questionable as the conditions used, i.e. serum depletion which causes the RGCs to die, are not reflecting the conditions of such diseases.

The present application discloses data showing that propentophylline has no effect on RGC survival when used in an assay that is based on RGCs dying due to complex I inhibition by rotenone, a condition well reflecting the pathology of many of the mitochondrial disorders described above (reviewed by Wong-Riley M., Energy Metabolism of the Visual System, Eye and Brain, 2010, 2 99-116).

Concluding from these negative effect of propentophylline, and following the argumentation of WO2000/032197, one would conclude that idebenone (compound IA) is expected to also show no effect in this disease relevant RGC survival assay, i.e. based on complex I inhibition. Instead, it was completely unexpected to find that idebenone - in sharp

contrast to propentophylline - is well able to strongly protect RGCs from cell death under conditions reflecting the pathological situation of mitochondrial dysfunction, thus further supporting the present invention.

Autosomal dominant optic atrophy (DOA) can be allocated as mitochondrial disease. Pathogenic OPA1 mutations cause autosomal dominant optic atrophy, a condition characterized by the preferential loss of retinal ganglion cells and progressive optic nerve degeneration. Approximately 20% of affected patients will also develop more severe neuromuscular complications, an important disease subgroup known as DOA(+). OPA1 was demonstrated to control both mitochondrial fusion and cristae morphology. In addition, OPA1 loss-of-function studies have shown that OPA1 also regulates apoptosis induction (Liesa M et al. Physiol Rev. 2009; 89:799-845). Although it is expressed in all the tissues assayed, OPA1 shows a specific tissue expression pattern, with the highest expression in the retina, brain, testis, liver, heart, skeletal muscle, and pancreas. Loss of OPA1 causes a marked reduction in mitochondrial membrane potential and a reduction in basal respiration and incapacity to enhance oxygen consumption in the presence of the uncoupler 2,4-dinitrophenol. Human fibroblasts from patients with certain OPA1 mutations (that cause autosomal dominant optic atrophy or ADOA), in addition to decreased rates of mitochondrial fusion, also show impaired ATP synthesis driven by complex I substrates. Due to their ability to enhance ATP synthesis the compounds of formula (I) are useful in the treatment of DOA.

Mitochondrial diseases also encompass various disorders of the human optic systems such as macular degeneration, glaucoma, retinopathy, cataract, optic disc drusen (ODD).

Although some macular dystrophies affecting younger individuals are sometimes described as macular degeneration, the term generally refers to age-related macular degeneration (AMD) and is a major cause of visual impairment in older adults (>50 years). Both for the inherited (VD) and acquired forms of macular degeneration, the major risk factors for this disorder are age, smoking, hypertension and diabetes. AMD, like LHON, is a medical condition which results in a loss of vision in the center of the visual field (the macula) because of damage to the retina. Although, changes in mitochondrial structure and function, such as loss of cristae and matrix density are a normal feature of ageing, these aberrations are significantly greater in AMD than in normal aging and also occur at an earlier time point (Feher J, Kovacs I, Artico M, Cavallotti C, Papale A, Balacco Gabrieli C. Neurobiol Aging. 2006 Jul;27(7):983-937), which would suggest that the severity of mitochondrial alterations are different between AMD and normal aging, and that the timing of damage to retinal cells may be critical for the development of AMD. Furthermore, AMD patients show increased mtDNA mutations selectively in the retina compared to blood cells while AMD- related changes in the mitochondrial proteome have also been reported. Consistent with mitochondrial involvement in the pathology of AMD, SanGiovanni et al. (2009) described multiple polymorphisms (SNPs) in mtDNA that were associated with AMD using a cohort of 215 patients with advanced AMD. These associations were driven entirely by the T2 haplogroup, and characterized by two variants in Complex I genes (A11812G of MT-ND4 and A14233G of MT-ND6), reminiscent of mutations in Complex I that cause LHON. Consequently, treatment of AMD patients with compounds that alter mitochondrial function (mitotropic) has been attempted (Feher J, Papale A, Mannino G, Gualdi L, Balacco Gabrieli C. Ophthalmologica. 2003 Sep-Oct;217(5):351-7), although the effects so far remained only marginal.

Glaucoma (optic neuropathy) is a disease in which the optic nerve is damaged, leading to progressive, irreversible loss of vision, which is often, but not always, associated with increased pressure of the fluid in the eye. The nerve damage involves loss of retinal ganglion cells in a characteristic pattern. There are many different sub-types of glaucoma but they can all be considered a type of optic neuropathy. Raised intraocular pressure is a significant risk factor for developing glaucoma. Untreated glaucoma leads to permanent damage of the optic nerve and resultant visual field loss, which can progress to blindness. Mitochondrial involvement in the pathology of glaucoma is supported by work from Guo (Guo Y, Johnson EC, Cepurna WO, Dyck J, Doser T, Morrison JC. Invest Ophthalmol Vis Sci. 2010 Nov 4. [Epub ahead of print]), who showed selective downregulation of mitochondrial genes and genes related to energy production in a rat model of glaucoma. Furthermore, similar to results described for macular degeneration, a higher prevalence of mtDNA alterations were also reported for glaucoma patients. Finally, overexpression of the mitochondrial protein OPA1, mutated in DOA, is protective in a mouse model of glaucoma (Ju WK, Kim KY, Duong-Polk KX, Lindsey JD, Ellisman MH, Weinreb RN. Increased optic atrophy type 1 expression protects retinal ganglion cells in a mouse model of glaucoma. Mol Vis. 2010 Jul 15;16:1331-42), which clearly demonstrates a causal relationship between mitochondrial dysfunction in acquired eye pathologies such as glaucoma. Mitochondrial dysfunction may either be due to nuclear or mitochondrial genes, by mechanical stress, insufficient blood supply, chronic hypoperfusion consequent to the commonly raised intraocular pressure in glaucomatous eyes, or by toxic xenobiotic).

Retinopathy is a general term that refers to some form of non-inflammatory damage to the retina of the eye. Next to the genetic or inherited forms of retinopathy, spontaneous forms can be induced by drugs, toxins and radiation (ionizing and ultra violet). Frequently, retinopathies are ocular manifestations of systemic diseases such as diabetes, hypertension, sickle cell disease or ciliopathy such as Bardet-Biedl syndrome. Similar to macular degeneration and glaucoma, mitochondrial involvement in retinopathy has been well described (reviewed by Jarrett SG, Lewin AS, Boulton ME. Ophthalmic Res. 2010;44(3):179-90, Lee S, Van Bergen NJ, Kong GY, Chrysostomou V, Waugh HS, O'Neill EC, Crowston JG, Trounce IA. Exp Eye Res. 2010 Aug 4. (Epub ahead of print).

A cataract is a clouding that develops in the crystalline lens of the eye or in its envelope, varying in degree from slight to complete opacity and obstructing the passage of light. The gradual yellowing and opacification of the lens may reduce the perception of blue colours. Cataracts typically progress slowly to cause vision loss and are potentially blinding if untreated. The condition usually affects both the eyes, but similar to the pathology of LHON and VD, one eye is almost always affected earlier than the other. As for the pathologies above, mitochondrial involvement in cataract formation is clearly described. Furthermore, cataract formation is directly associated with some mitochondrial disorders such as dominant optic atrophy with cataracts, autosomal dominant progressive external ophthalmoplegia (PEOA3), PEOA2, mitochondrial myopathy caused by mutations of COX II or various others genes, Sengers syndrome, as well as mutations of mitochondrial genes such as mitochondrial tRNA-Ser (MTTS2), GFER, OPA3 but also large mitochondrial deletions.

Optic disc drusen (ODD) or optic nerve head drusen (ONHD) are globules of mucoproteins and mucopolysaccharides that progressively calcify in the optic disc. They are thought to be the remnants of the axonal transport system of degenerated retinal ganglion cells. ODD have also been referred to as congenitally elevated or anomalous discs, pseudopapilledema, pseudoneuritis, buried disc drusen, and disc hyaline bodies. They are associated with vision loss of varying degree occasionally resulting in blindness. Mitochondrial impairment due to $Ca^{2+}$ overload has been suggested in the process of drusen formation (Tso MO. Ophthalmology. 1981 Oct;88(10):1066-80). Furthermore, in the case of age-related maculopathy, higher risk for soft drusen were also found to be associated with mitochondrial haplotype J, whereas haplotype H was associated with significantly lower risk (Jones MM, Manwaring N, Wang JJ, Rochtchina E, Mitchell P, Sue CM. Arch Ophthalmol. 2007 Sep;125(9):1235-40).

Thus, Chrysostomou et al. (Chrysostomou V, Trounce IA, Crowston JG. Ophthalmic Res. 2010;44(3): 173-8) suggested that mitochondrial dysfunction, inherited or as a cause or consequence of injury, renders ocular cells (in particular retinal ganglion cells) sensitive to degeneration. Therapeutic approaches that target mitochondria should therefore provide a general means of protecting lens and retinal ganglion cells from degeneration, regardless of the etiology of the disease.

The compounds of formula (I) *may be* also useful for treating psychiatric disorders, metabolic disorders, cancer, multiple sclerosis, primary progressive multiple sclerosis, or immune dysfunction, as will be detailed in the following.

In type II diabetes a clear connection between excess production of oxygen radicals, mitochondrial dysfunction and disease progression has been established [reviewed by Friederich M, Hansell P, Palm F Curr Diabetes Rev. 2009 May;5(2):120-44], while in a wider sense most pathologies of metabolic syndrome such as heart failure can also be attributed to mitochondrial dysregulation [reviewed by Bugger H, Abel ED. Clin Sci (Lond). 2008 Feb;114(3):195-210].

Under conditions where cellular energy production is reduced, such as during ageing-related decline of mitochondrial function [Boumezbeur F, Mason GF, de Graaf RA, Behar KL, Cline GW, Shulman GI, Rothman DL, Petersen KF J Cereb Blood Flow Metab. 2009 Sep 30] or during strenuous activity, it is beneficial to raise the production of ATP to counteract anticipated cell loss or toxicity from energy deprivation. Contrary to that, under special conditions, such as obesity, it can also be beneficial to restrain the mitochondrial capacity for ATP production to aide therapeutic weight loss efforts.

Surprisingly, it was found that the compounds of formula (I) have an effect on body weight gain, possibly due to an effect on ATP production, which could be demonstrated *in vitro.* Thus, there is a strong indication that the compounds of formula (I) show a beneficial effect in the treatment of diabetes type II, obesity and metabolic syndrome.

While modulators of mitochondrial function have been suggested as anti-cancer agents mainly via an apoptosis-inducing function, recent data also links the metastatic potential of tumours directly to mitochondrial DNA and mitochondrial radical production [reviewed by Ishikawa K, Koshikawa N, Takenaga K, Nakada K, Hayashi J Mitochondrion. 2008;8(4):339-44]. Therefore; modulating mitochondria in this context could not only be employed as anti-cancer strategy but also to prevent metastasis of malignancies which makes the compounds according to formula (I) useful as cancer therapeutics.

Altered mitochondrial metabolism is also linked to multiple dysfunctions of the immune system. In patients with systemic juvenile idiopathic arthritis for example, reduced expression of mitochondrial respiratory chain genes was observed [Ishikawa S, Mima T, Aoki C, Yoshio-Hoshino N, Adachi Y, Imagawa T, Mori M, Tomiita M, Iwata N, Murata T, Miyoshi M, Takei S, Aihara Y, Yokota S, Matsubara K, Nishimoto N Ann Rheum Dis. 2009;68(2):264-72]. Oxidative stress and mitochondrial pathology has been reported to be associated with sepsis [Victor VM, Espulgues JV, Hernández-Mijares A, Rocha M Infect Disord Drug Targets. 2009;9(4):376-89]. Furthermore, targeting mitochondrial function has been suggested as treatment strategy for several autoimmune disorders such as arthritis and psoriasis [J.J. Bednarski, R.E. Warner, T. Rao, F. Leonetti, R. Yung, B.C. Richardson, K.J. Johnson, J.A. Ellman, A.W. Opipari Jr., and G.D. Glick Arthritis & Rheumatism, 2003;48, 757] and could be applied to modulate multiple immunological endpoints such as microbial infection [reviewed by Arnoult D, Carneiro L, Tattoli I, Girardin SE Semin Immunol. 2009;21 (4):223-32] and allergy [Chodaczek G, Bacsi A, Dharajiya N, Sur S, Hazra TK, Boldogh I. Mol Immunol. 2009;46(13):2505-14]. Thus, the compounds of formula (I) are also useful in the treatment of arthritis, psoriasis, sepsis, allergy and microbial infections.

Regarding the treatment of multiple sclerosis (MS), the first connection between mitochondria and multiple sclerosis was established due to the finding of an MS-like disease in a subset of patients with Leber's hereditary optic

neuropathy (LHON), a disease caused by mutations in mitochondrial (mt)DNA (Olsen NK, et al. Acta Neurol. Scand. 1995; 91:326-329). Similar to findings from patients with mitochondrial disease, elevated levels of metabolites from extra-mitochondrial glucose metabolism have been found in the cerebrospinal fluid (CSF) of MS patients and correlated with disease progression (Regenold WT et al. J. Neurol. Sci. 2008; 275:106-112). As extra-mitochondrial glucose metabolism increases with impaired mitochondrial glucose metabolism (Seyama K et al. Acta Neurol. Scand. 1989; 80:561-568), this implicates mitochondrial dysfunction in MS disease progression. Moreover, mitochondria in neuronal cell bodies in non-demyelinated MS grey matter were found to have decreased activity of complexes I and III, and, as neurons partly provide their axon with mitochondria, this might further impair mitochondrial function in chronically demyelinated axons (Dutta, R. et al. Ann. Neurol. 2006; 59:478-489). Thus, the compounds of formula (I) are useful in the treatment of multiple sclerosis.

The vast majority of newly-diagnosed MS patients develop the relapsing-remitting form of the disease (RR-MS), in which periods of neurological worsening are followed by periods of spontaneous remission, at least at the beginning of the disease process. About 10-15% of patients develop primary progressive MS (PP-MS), characterized by progressive accumulation of neurological disability from the disease onset, without any superimposed worsening (i.e. relapses) or improvements (remissions).

Importantly, primary progressive MS (PP-MS) patients differ from RR-MS patients in several important characteristics: They tend to be older at the time of disease onset (mean 40 vs. 30 years); males and females tend to be affected equally; clinically there is a high prevalence of cortico-spinal dysfunction characterized by progressive weakness and spasticity; patients have more prominent involvement of the spinal cord and generally lower amount of distinct white matter lesions (i.e. plaques) in the brain and less evidence for brain inflammatory activity and, most importantly, PP-MS patients do not respond to immunomodulatory therapies with proven efficacy in RR-MS. Both new imaging modalities and pathological data suggest that in PP-MS, CNS pathology is more diffuse and occurs to some extent independently of focal lesions.

There are currently no treatments with proven therapeutic efficacy for PP-MS. Surprisingly, it has been found that the compounds of formula (I) which are effective as modulators of mitochondrial diseases are able to alleviate the symptoms of PP-MS.

Preparation

As stated elsewhere in the application, preparation methods for the compounds of formula (I) are described in the patent literature as well as in the scientific literature.

With respect to the preparation of Compound ID, the general method described in EP-A-0038674 for the preparation of the corresponding carboxylic acid (COOH instead of $CH_2OH$) cannot be used for the preparation of compound ID as the reduction of the carboxyl function would lead to a simultaneous reduction of the quinone system, too. An analogue with a hexamethylene chain instead of a decamethylene is described in US4139545 (Takeda), which is cited in EP-A-0038674. However, this route turned out to deliver unsatisfying results in terms of work-up of the reaction mixtures and yields. It has been found that Compound ID can be prepared by the following novel preparation method (Scheme 1).

**Scheme 1**

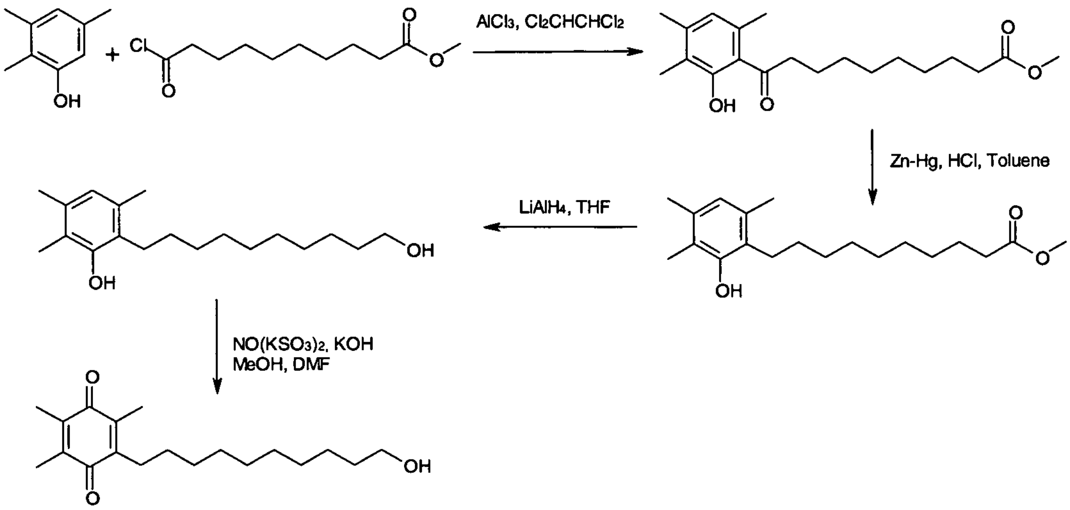


As can be taken from Scheme 1, 2,3,5-trimethylphenol is reacted with methyl sebacoyl chloride in the presence of a catalyst such as aluminum chloride in a solvent such as 1,1,2,2-tetrachloroethane (step 1) followed by a reduction with a reducing agent such as amalgamated zinc in a solvent such as toluene (step 2). The obtained ester is reduced with a reagent such as lithium aluminum hydride in a solvent such as tetrahydrofurane (step 3). Reacting with potassium nitrosodisulfonate in the presence of a base such a potassium hydroxide yields the final Compound ID (step 4).

Salts

The term "pharmaceutically acceptable salts" refers to salts prepared from pharmaceutically acceptable non-toxic bases or acids including inorganic or organic bases and inorganic or organic acids. Salts derived from inorganic bases include aluminum, ammonium, calcium, copper, ferric, ferrous, lithium, magnesium, manganic salts, manganous, potassium, sodium, zinc and the like. Particularly preferred are the ammonium, calcium, lithium, magnesium, potassium and sodium salts. Salts derived from pharmaceutically acceptable organic non-toxic bases include salts of primary, secondary and tertiary amines, substituted amines including naturally occurring substituted amines, cyclic amines and basic ion exchange resins, such as arginine, betaine, caffeine, choline, N,N'-dibenzylethylenediamine, diethylamine, 2-diethylaminoethanol, 2-dimethylamino-ethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, salts may be prepared from pharmaceutically acceptable non-toxic acids, including inorganic and organic acids. Such acids include acetic, benzenesulfonic, benzoic, camphorsulfonic, citric, ethanesulfonic, formic, furnaric, gluconic, glutamic, hydrobromic, hydrochloric, isethionic, lactic, maleic, malic, mandelic, methanesulfonic, malonic, mucic, nitric, parnoic, pantothenic, phosphoric, propionic, succinic, sulfuric, tartaric, p-toluenesulfonic, trifluoroacetic acid and the like. Particularly preferred are citric, fumaric, hydrobromic, hydrochloric, maleic, phosphoric, sulfuric and tartaric acids.

It will be understood that, as used herein, references to the compounds of formula (I) are meant to also include the pharmaceutically acceptable salts.

Administration

The compounds of formula (I) are preferably formulated into a dosage form prior to administration. Accordingly the present invention also includes a pharmaceutical composition comprising a compound of formula (I) or a pharmaceutically acceptable salt or solvate thereof and a suitable pharmaceutical carrier.

The carrier(s) must be "acceptable" in the sense of being compatible with the other ingredients of the composition and not deleterious to the recipient thereof. Proper formulation is dependent upon the route of administration chosen. Any of the well-known techniques, carriers, and excipients may be used as suitable and as understood in the art; e.g., in Remington's Pharmaceutical Sciences. The pharmaceutical compositions of the present invention may be manufactured in a manner that is itself known, e.g., by means of conventional mixing, dissolving, granulating, dragee-making, levigating, emulsifying, encapsulating, entrapping or compression processes.

Any suitable route of administration may be employed for providing a mammal, especially a human with an

effective dosage of a compound of formula (I).

Suitable administration routes include oral, parenteral (including subcutaneous, intradermal, intramuscular, intravenous, intraarticular, and intramedullary), intraperitoneal, transmucosal, transdermal, rectal and topical (including dermal, buccal, sublingual and intraocular) administration although the most suitable route may depend upon for example the condition and disorder of the recipient.

In a preferred embodiment in combination with any of the above or below embodiments, the administration route is oral. In another preferred embodiment in combination with any of the above or below embodiments, the administration route is topical ocular. In another preferred embodiment in combination with any of the above or below embodiments, the administration route is an intraocular injection. In another preferred embodiment in combination with any of the above or below embodiments, the administration route is an intraocular depot implant.

## Formulation and Dose Ranges

The formulations may conveniently be presented in unit dosage form and may be prepared by any of the methods well known in the art of pharmacy. All methods include the step of bringing into association a compound of the present invention or a pharmaceutically acceptable salt or solvate thereof ("active ingredient") with the carrier which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient. The active ingredient may further be presented as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

Pharmaceutical preparations which can be used orally include tablets, push-fit capsules made of gelatin, as well as soft, sealed capsules made of gelatin and a plasticizer, such as glycerol or sorbitol. Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the the powdered compound moistened with an inert liquid diluent. The tablets may optionally be coated or scored and may be formulated so as to provide slow or controlled release of the active ingredient therein.

All formulations for oral administration should be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

The compounds of the present invention may be formulated for parenteral administration by injection, e.g. as intraocular, intraveneous, subcutaneous, intramuscular, or intraarterial injection. The injection may be administered *by* bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The formulations may be presented in unit-dose or multi-dose containers, for example sealed ampoules and vials, and may be stored in powder form or in a freeze-dried (lyophilized) condition requiring only the addition of the sterile liquid carrier, for example, saline or sterile pyrogen-free water, immediately prior to use. Extemporaneous injection solutions and suspensions may be prepared from sterile powders, granules and tablets of the kind previously described.

Formulations for parenteral administration include aqueous and non-aqueous (oily) sterile injection solutions of the active compounds which may contain antioxidants, buffers, bacteriostats and solutes which render the formulation isotonic with the blood of the intended recipient; and aqueous and non-aqueous sterile suspensions which may include suspending agents and thickening agents. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of

the compounds to allow for the preparation of highly concentrated solutions.

In addition to the formulations described previously, the compounds may also be formulated as a depot preparation. Such long acting formulations may be administered by implantation (for example intraocular or subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For buccal or sublingual administration, the compositions may take the form of tablets, lozenges, pastilles, or gels formulated in conventional manner. Such compositions may comprise the active ingredient in a flavoured basis such as sucrose and acacia or tragacanth.

The compounds may also be formulated in rectal compositions such as suppositories or retention enemas, e.g., containing conventional suppository bases such as cocoa butter, polyethylene glycol, or other glycerides.

Compounds of the present invention may be administered topically, that is by non-systemic administration. This includes the application of a compound of the present invention externally to the epidermis or the buccal cavity and the instillation of such a compound into the ear, eye and nose, such that the compound does not significantly enter the blood stream. In contrast, systemic administration refers to oral, intravenous, intraperitoneal and intramuscular administration.

Formulations suitable for topical administration include liquid or semi-liquid preparations suitable for penetration through the skin to the site of inflammation such as gels, liniments, lotions, creams, ointments or pastes, and drops suitable for administration to the eye, ear or nose.

In a topical ocular formulation, the amount of compound of formula (I) will generally be in the range of 0.001 to 10% weight/volume (%w/v). Preferred concentrations range from 0.1 to 5 %w/v. Topical administration to the eye is given one to six times per day. A typical formulation contains 0.1 - 5 % of compound of formula (I), 0.5 %w/v hydroxypropylmethylcellulose (HMPC), 0.8 %w/v sodium chloride, 0.28 %w/v sodium phosphate, 0.01 %w/v edetate disodium, 0.01 %w/v benzalkonium chloride. The pH is adjusted to 7.2 - 7.4. Purified water is added q.s.

Gels for topical or transdermal administration of compounds of the subject invention may comprise, generally, a mixture of volatile solvents, nonvolatile solvents, and water. The volatile solvent component of the buffered solvent system may preferably include lower ($C_1$-$C_6$) alkyl alcohols, lower alkyl glycols and lower glycol polymers. More preferably, the volatile solvent is ethanol. The volatile solvent component is thought to act as a penetration enhancer, while also producing a cooling effect on the skin as it evaporates. The nonvolatile solvent portion of the buffered solvent system is selected from lower alkylene glycols and lower glycol polymers. Preferably, propylene glycol is used. The nonvolatile solvent slows the evaporation of the volatile solvent and reduces the vapor pressure of the buffered solvent system. The amount of this nonvolatile solvent component, as with the volatile solvent, is determined by the pharmaceutical compound or drug being used. When too little of the non-volatile solvent is in the system, the pharmaceutical compound may crystallize due to evaporation of volatile solvent, while an excess will result in a lack of bioavailability due to poor release of drug from solvent mixture.

The buffer component of the buffered solvent system may be selected from any buffer commonly used in the art; preferably, water is used. There are several optional ingredients which can be added to the topical composition. These include, but are not limited to, chelators and gelling agents. Appropriate gelling agents can include, but are not limited to, semisynthetic cellulose derivatives (such as hydroxypropylmethylcellulose) and synthetic polymers, and cosmetic agents.

Lotions according to the present invention include those suitable for application to the skin or eye. An eye lotion may comprise a sterile aqueous solution optionally containing a bactericide and may be prepared by methods similar to those for the preparation of drops. Lotions or liniments for application to the skin may also include an agent to hasten drying and to cool the skin, such as an alcohol or acetone, and/or a moisturizer such as glycerol or an oil such as castor oil or arachis oil.

Creams, ointments or pastes according to the present invention are semi-solid formulations of the active ingredient for external application. They may be made by mixing the active ingredient in finely-divided or powdered form, alone or in solution or suspension in an aqueous or non-aqueous fluid, with the aid of suitable machinery, with a greasy or non-greasy base. The base may comprise hydrocarbons such as hard, soft or liquid paraffin, glycerol, beeswax, a metallic soap, a mucilage; an oil of natural origin such as almond, corn, arachis, castor or olive oil, wool fat or its derivatives or a fatty acid such as steric or oleic acid together with an alcohol such as propylene glycol or a macrogel. The formulation may incorporate any suitable surface active agent such as an anionic, cationic or non-ionic surfactant such as a sorbitan ester or a polyoxyethylene derivative thereof. Suspending agents such as natural gums, cellulose derivatives or inorganic materials such as silicaceous silicas, and other ingredients such as lanolin, may also be included.

Drops according to the present invention may comprise sterile aqueous or oily solutions or suspensions and may be prepared by dissolving the active ingredient in a suitable aqueous solution of a bactericidal and/or fungicidal agent and/or any other suitable preservative, and preferably including a surface active agent.

Formulations for topical administration in the mouth, for example buccally or sublingually, include lozenges

comprising the active ingredient in a flavored basis such as sucrose and acacia or tragacanth, and pastilles comprising the active ingredient in a basis such as gelatine and glycerine or sucrose and acacia.

For administration by inhalation the compounds according to the invention are conveniently delivered from an insufflator, nebulizer pressurized packs or other convenient means of delivering an aerosol spray. Pressurized packs may comprise a suitable propellant such as dichlorodifluoromethane, trichlorofluoromethane, dichlorotetra-fluoroethane, carbon dioxide or other suitable gas. In the case of a pressurized aerosol, the dosage unit may be determined by providing a valve to deliver a metered amount. Alternatively, for administration by inhalation or insufflation, the compounds according to the invention may take the form of a dry powder composition, for example a powder mix of the compound and a suitable powder such as lactose or starch. The powder composition may be presented in unit form, in for example, capsules, cartridges, gelatine or blister packs from which the powder may be with the aid of an inhalator or insufflator.

Preferred unit dosage formulations are those containing an effective dose, as herein below recited or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavoring agents.

The compounds of the present invention may be administered via any route discussed above at a dose range for adult humans which is generally from 0.01 mg/kg/day to 60 mg/kg/day, more preferably from 0.01 mg/kg/day to 30 mg/kg/day, most preferably from 0.01 mg/kg/day to 15 mg/kg/day.

The amount of active ingredient that may be combined with the carrier materials to produce a single dosage form will vary depending upon the host treated and the particular mode of administration.

The compounds of the subject invention can be administered in various modes, e.g. orally, topically, or by injection. The precise amount of compound administered to a patient will be the responsibility of the attendant physician. The specific dose level for any particular patient will depend upon a variety of factors including the activity of the specific compound employed, the age, body weight, general health, sex, diets, time of administration, route of administration, rate of excretion, drug combination, the precise disorder being treated, and the severity of the indication or condition being treated. Also, the route of administration may vary depending on the condition and its severity.

**Analytical LC-MS**

The compounds of formula (I) were analyzed by analytical LC-MS. The conditions are summarized below.

<u>Analytical conditions summary:</u>

LC10Advp-Pump (Shimadzu) with SPD-M10Avp (Shimadzu) UV/Vis diode array detector and QP2010 MS-detector (Shimadzu) in ESI+ modus with UV-detection at 214, 254 and 275 nm,
Column: Waters XTerra MS C18, 3.5 μm, 2.1 * 100 mm,
linear gradient with acetonitrile in water (0.15% HCOOH)
Flow rate of 0,4 ml/min;

| | |
|---|---|
| Mobile Phase A: | water (0.15% HCOOH) |
| Mobile Phase B: | acetonitrile (0.15% HCOOH) |

Methods are:
**A:**
<u>Polar QC linear gradient;</u>
start concentration 1% acetonitrile

| | | |
|---|---|---|
| 9.00 | B.Conc | 30 |
| 10.00 | B.Curve | 3 |
| 12.00 | B.Conc | 99 |
| 15.00 | B.Conc | 99 |
| 15.20 | B.Conc | 1 |
| 18.00 | Pump STOP | |

**B:**
<u>Medium QC linear gradient;</u>

start concentration 10% acetonitrile

| | | |
|---|---|---|
| 10.00 | B.Conc | 60 |
| 11.00 | B.Curve | 2 |
| 12.00 | B.Conc | 99 |
| 15.00 | B.Conc | 99 |
| 15.20 | B.Conc | 10 |
| 18.00 | Pump STOP | |

**C:**
Unpolar QC linear gradient;
start concentration 15% acetonitrile

| | | |
|---|---|---|
| 12.00 | B.Conc | 99 |
| 15.00 | B.Conc | 99 |
| 15.20 | B.Conc | 15 |
| 18.00 | STOP 0 | |

**B.Curve** Set the gradient curve of B (C, D) liquid in the gradient mode. The curve at
**C.Curve** 0<=t<=T can be calculated by the following equation, where c(t) is the
**D.Curve** concentration of the liquid B (C, D) at the relative time t in each interval, T is the gradient time, and a is the value for [B(C,D). Curve].

1. **a>0**

$$C(t)=C1+(C2-C1)*\{ (exp(a*t/T)-1) / (exp(a)-1$$

2. **a=0**(line)

$$C(t)=C1+(C2-C1)*(t/T)$$

3. **a<0**

$$C(t)=C1+(C2-C1)*\{ (exp\{|a|\ (1-t/T)\}-1) / (exp|a|-1)\ \}$$

C1:Initial value of [B(C,D).Conc]
C2:Final value of [B(C,D).Conc] (To be set by LC Program)
-10 - 10

**Examples**

**Synthesis of Compound ID:**

**Intermediate IDa):**

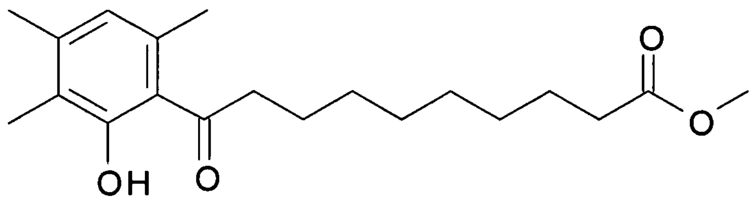

To a solution of 2,3,5-trimethylphenol (0.5 g, 1 eq) in 1,1,2,2-tetrachloroethane (15 ml) was added a solution of aluminum chloride (1.22 g, 2.5 eq) and methyl sebacoyl chloride (1.03 g, 1.2 eq) in 1,1,2,2-tetrachloroethane (5 ml) at 0°C under argon atmosphere. The reaction mixture was stirred at 140°C for 24h and was hydrolyzed at 0°C with water (10 ml) and a 2N hydrochloric acid solution (10 ml). The solution was extracted with ethyl acetate (20 ml). After phase separation, the aqueous layer was extracted with ethyl acetate (2 X 20 ml), the organic layers were combined and the solvents were removed under reduced pressure. The crude product was purified by flash-chromatography (ethyl acetate-cyclohexane).

**Intermediate IDb):**

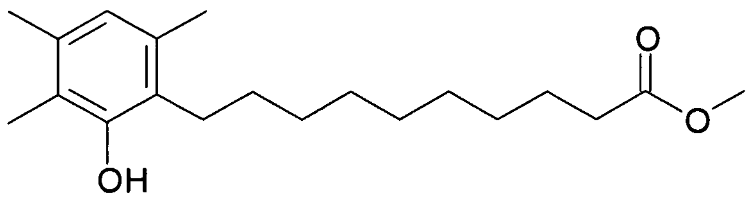

In a round bottomed flask, zinc (13 g, 11 eq), mercury chloride (1.3 g, 0.26 eq), water (15 ml) and conc. hydrochloric acid (0.15 ml) were stirred for 5 min and the solution was decanted. The zinc was washed with water and transferred into a new clean round bottomed flask. To this amalgamated zinc were added water (2 ml), conc. hydrochloric acid (10 ml), toluene (10 ml) and intermediate IDa (6 g, 1 eq). The reaction mixture was stirred vigorously at room temperature for 10 minutes and at 140 °C for 3 days. The same amount of amalgamated zinc as before was added and the reaction mixture was stirred for 3 additional days at 140 °C. The reaction mixture was transferred into a separating funnel, and the obtained organic layer was transferred into a new clean round bottomed flask. To this reaction mixture were added water (0.5 ml), conc. hydrochloric acid (2 ml), and freshly activated zinc (2 g). The reaction mixture was stirred for 2 additional days at 140°C. The solution was decanted from the un-reacted zinc and was extracted twice with ethyl acetate. The combined organic extract was washed with 0.1 M aqueous hydrochloric acid solution (25 ml), dried over sodium sulfate, filtrated, and volatiles were removed under reduced pressure. The crude product was purified by flash chromatography (ethyl acetate -cyclohexane).

**Intermediate IDc):**

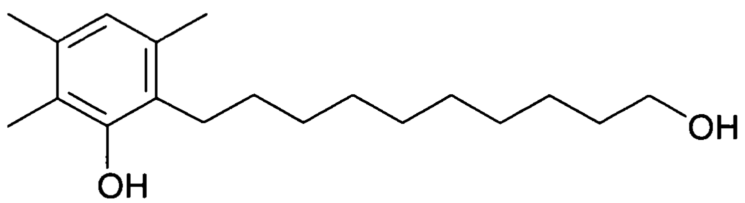

Intermediate IDb (3.7 g, 1 eq) was dissolved in tetrahydrofuran (100 ml) and lithium aluminum hydride (0.26 g, 1.1 eq) was added at 0 °C under argon atmosphere. The reaction mixture was stirred at 0°C for 10 minutes until to obtain a complete conversion. The solution was poured onto a saturated ammonium chloride solution (200 ml) at 0 °C and ethyl acetate (200 ml) was added. After phase separation, the aqueous phase was extracted with ethyl acetate (150 ml). The combined organic layers were dried over sodium sulfate, filtrated and solvents were removed under reduced

pressure. The crude product was purified by flash-chromatography (ethyl acetate-cyclohexane).

**Example ID:**

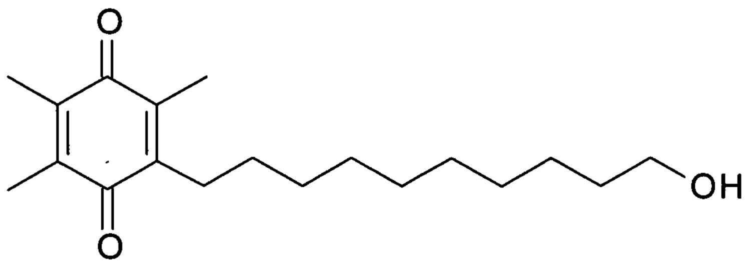

Intermediate IDc (1.62 g, 1 eq) was dissolved in a mixture of methanol (50 ml) and *N,N*-dimethylformamide (50 ml). To this solution, a solution of 0.5% of potassium hydroxide (80 ml) and potassium nitrosodisulfonate (4 g, 3 eq) were added and the reaction mixture was stirred at room temperature for 14h until to obtain a complete conversion. A 1M aqueous hydrochloric acid solution (100 ml) was added and the solution was extracted with ethyl acetate (2 X 200 ml). The combined organic layers were dried over sodium sulfate, filtrated, and volatiles were removed under reduced pressure. The crude product was purified first by flash-chromatography (ethyl acetate -cyclohexane) and then with preparative HPLC-MS.
LCMS: method C, $t_R$ (min) = 8.86, MW (calc.) = 306.44, $[M+H]^+$ (found) = 307.

**Biological Assays:**

*A. Cytotoxicity Assay*

This assay investigates cytotoxicity which is defined as the cell-killing property of a chemical compound, independent from the mechanisms of cell death. The HepG2 hepatoblastoma cell line is one of the most common human cell lines for hepatotoxicity studies. Even though the cells lack a part of the metabolizing enzymes present in fresh hepatocytes, they have been shown to be a useful tool for studying the toxicity of hepatotoxins. This assay measures metabolic activity of living cells using the WST-1 cell proliferation reagent (Roche Diagnostics). The tetrazolium salt WST-1 is cleaved to water soluble formazan by cellular enzymes. An expansion in the number of viable cells results in an increase in the overall activity of mitochondrial dehydrogenases in the sample. This augmentation in enzyme activity leads to an increase in the amount of formazan dye formed, which directly correlates to the number of metabolically active cells in the culture. The formazan dye produced by metabolically active cells is directly quantified in a scanning multiwell spectrophotometer by measuring the absorbance of the formazan dye solution between 420 and 480 nm. Healthy HepG2 cells, when maintained in culture, continuously divide and multiply over time. A toxic chemical, regardless of site and mechanism of action, will interfere with this process and result in the reduction of the growth rate reflected in the cell number.

*Experimental Procedure:* Hep-G2 cells are seeded into a 96-well microplate and maintained in culture for 24 hours. They are then exposed to the test compound over a range of eight concentrations. After 24 hours exposure, the cells are incubated in presence of reagent WST-1 for 30 min before measuring the absorbance of the formazan dye formed. Cytotoxicity is expressed as a concentration dependent reduction of the conversion formazan dye formation due to a decrease in cell proliferation as compared to untreated cells. The $TC_{50}$ (Toxic Concentration 50%), is defined as the concentration of tested compound required to reduce the cellular viability by 50% relative to the control culture. The lower the $TC_{50}$ value, the higher is the cytotoxic potential of the test compound. The TC 50% values were calculated using the following formula:

$$TC_{50} = Conc._{>50} - \frac{(Conc._{>50} - Conc_{<50}) \cdot (\%_{>50} - 50)}{(\%_{>50} - \%_{<50})}$$

a) Conc. > 50 = maximum measured concentration with the % of solvent control > 50 %
b) Conc. < 50 = minimum measured concentration with the % of solvent control < 50 %
c) % > 50 = relative absorbance at a) in %

d) % < 50 = relative absorbance at b) in %

Table 1

| *Compound* | *$TC_{50}$ [μM]* |
|---|---|
| ***Compound IA*** | ***100*** |
| ***Compound IC*** | ***71.94*** |
| ***Compound IE*** | ***100*** |
| ***Compound IF*** | ***100*** |

As can be taken from Table 3, compounds of formula (I) clearly demonstrate no toxicity in the dose range evaluated.

*B. ATP Assay*

Electrons donated from the citric acid cycle in form of NADH are transferred between complexes I, II and III of the respiratory chain. In this process mitochondrial membrane potential is generated through proton pumps in the mitochondrial inner membrane. This electrochemical gradient across the inner mitochondrial membrane is used in healthy cells to provide the energy to drive mitochondrial ATP production. Consequently, cellular ATP levels are a good indicator of mitochondrial function. In multiple states of disease (i.e. Alzheimer's-, Parkinson's-, Huntington's-disease and mitochondrial disorders) this mode of ATP production is impaired and alternative modes of ATP production are utilized that can be associated with toxic byproducts (i.e. lactic acidosis due to increased anaerobic glycolysis). There is a general consensus that especially in tissues with high energy demand such as muscle and brain, chronic reduction of ATP levels ultimately leads to cell death. Therefore, compounds that significantly reduce ATP levels under normal growth conditions *in vitro* can be regarded as harbouring some potential for toxicity. However, some compounds have been described to exert positive effects on life span and pathological endpoints such as oxidative stress despite a reduction of energy production (Onken & Driscoll 2010). A potential inhibition of mitochondrial respiration can be detected through special growth conditions *in vitro.* C can distinguish between mitochondrial and non-mitochondrial ATP production (i.e. glycolysis), since under conditions of low glucose, ATP is mainly produced by oxidative phosphorylation.

*Experimental Procedure:* This assay was therefore performed in the absence of glucose in the medium. It has to be noted, however, that low levels of glucose contained in the serum result in estimated glucose concentrations of approximately 0.5mM. We therefore refer to this condition as "low glucose". Immortalized lymphoblastoid cells (BC1 LCL) were used to characterize the effect of compounds on ATP levels under different glucose levels by an ATP dependent luciferase reaction. Briefly, cells were seeded in a 24-well plate at a density of $5*10^5$ cells/ml with 1 ml per well in two different media containing 25 mM or no glucose, respectively. Both media were supplemented with 10% fetal bovine serum, 1% Pen/Strep, 200 mM L-glutamine. Cells were treated with 0.1% (v/v) of compounds (10 mM in DMSO; final concentration: 10 μM) and incubated for 72 h at 37 °C, 5% $CO_2$, and 90% rH. The number of cells was counted and after brief washing in PBS and collecting through centrifugation (5 min; 200 x g) the cells were lysed in a volume of 0.5 ml lysis solution (4 mM EDTA, 0.2% Triton X-100) for 15 min.on ice and 10 μl of lysate was added into a white 96 well plate. In parallel, ATP standards (concentrations: 0, 1, 2, 4, 6, 8, and 12 μM in PBS) were also added into the 96 well plate. The reaction was started by addition of 100 μl reaction mix (300 μM D-Luciferin, 5 μg/ml firefly luciferase, 75 μM DTT, 25 mM HEPES, 6.25 mM $MgCl_2$, 625 μM EDTA and 1 mg/ml BSA) and the luminescence signal was quantified in a multimode plate reader (Tecan M1000 plate reader; luminescence integration time: 100 ms). The concentration of cellular ATP normalized to cell number is calculated for each well and triplicate measurements are averaged. The ATP levels are given in Table 2.

Table 2

| *Compound* | *ATP levels [%]* |
|---|---|
| ***Compound IC*** | ***63*** |
| ***Compound IE*** | ***95*** |

In general, under normal growth conditions, more ATP is beneficial for the cell. However, under low glucose conditions, a treatment-dependent reduction of ATP levels can indicate an inhibition of mitochondrial respiration, which mimics calory restriction and is therefore likely to initiate all positive effects associated with calory restriction, such as longevity (Copeland et al. Current Biol 2009; 19:1-8). Nevertheless, it needs to be ascertained that no toxicity or other adverse effects such as ROS production are associated with this reduced respiration.

*C: Lipid Peroxidation Assay*

Lipid peroxidation is a well defined mechanism of cellular damage in both animals and plants that occurs during aging and in some disease states. This process proceeds by a free radical chain reaction mechanism. It most often affects polyunsaturated fatty acids, because they contain multiple double bonds in between which methylene-CH2-groups possess especially reactive hydrogens. If not terminated fast enough, lipid peroxidation damages cellular membranes, affect membrane fluidity and also mitochondrial function. In addition, end products of lipid peroxidation may be mutagenic and carcinogenic. For instance, the reactive end product of lipid peroxidation, malondialdehyde, directly causes DNA damage.

*Experimental Procedure:* The fluorescent BODIPY® (4,4-difluoro-3a,4adiaza-s-indacene) fluorophore is an effective tracer of lipid trafficking, as well as being useful general-purpose membrane probes. BODIPY 581/591-C11 can be used to measure antioxidant activity in lipid environments by exploiting its loss of fluorescence upon interaction with peroxyl radicals. Primary human fibroblasts C4 (GM04545, Coriell) (passage <12) were seeded at a concentration of 2000 cells/ well into black 96 well plates and incubated for 72 hours in DMEM in the presence of 10 μM test compound or DMSO only. After that, 0.1 ml freshly prepared dye solution (HBSS, containing BODIPY dye 1:1000 from stock solution) was added and cells were returned to the incubator for 30 min. After 2 brief washes with 0.1 ml warmed PBS fluorescence was measured in 50 μl PBS. Fluorescence for 4 individual areas per well were individually quantified at two wavelengths ($Ex_1$: 490, $Em_1$: 600; $Ex_2$: 490, $Em_2$: 530, bandwith 10nm, 50 flashes, 400Hz frequency, 20 μs integration time).

The extent of lipid peroxidation of various compounds of the present invention is shown below in Table 3.

Table 3

| *Compound* | *Lipid peroxidation [%]* |
|---|---|
| ***Compound IA*** | ***98*** |
| ***Compound IC*** | ***92*** |
| ***Compound ID*** | ***110*** |
| ***Compound IE*** | ***110*** |
| ***Compound IF*** | ***93*** |

Altered mitochondrial function, such as in mitochondrial disorders, can lead to cellular damage via lipid peroxidation. Altering mitochondrial function through small molecules therefore also has the inherent risk of producing lipid peroxidation. In this context, all compounds of Table 3 that leave basal lipid peroxidation levels unchanged (around 100%) do not show any major inherent toxic liability as demonstrated by the data included in Table 1.

*D: Measurement of mitochondrial complex II activity*

Complex II (CII) is an enzyme complex bound to the matrix face of the inner mitochondrial membranes. It consists of four subunits with several different enzymatic activities. One of these is the citric acid-cycle enzyme succinate dehydrogenase, which catalyzes the conversion from succinate to fumarate. In this reaction electrons are transferred from succinate to the prosthetic group FAD thus generating $FADH_2$ (Ackrell 2000). These electrons are then transferred from the reduced $FADH_2$ to ubiquinone, from ubiquinone to the reaction centers of complex III (CIII), and finally to the cytochrome c. During this electron translocation process, complex III pumps four protons from matrix to the intermembrane space. These proton fluxes can be detected as electrical currents on SSM-based SURFE2R sensors (http://www.sd-heidelberg.de/index.html). In the SURFE2R CII-CIII assay, both, CII and CIII are activated simultaneously by first equilibrating the sensors with succinate and then activating the CII-CIII reaction by oxidized cytochrome c. The resulting efflux of protons out from the membrane compartment induces negative currents, which are sensitive to the CII and CIII inhibitors malonate and antimycin A, respectively.

*Experimental Procedure:* SURFE2R SSM sensors were coated with inner mitochondrial membranes according to the standard protocols. Briefly, sensors were filled with 50 μL of SensorPrep A solution and incubated for 10-15 min. Afterwards the solution was removed, sensor were rinsed with deionized water three times dried in a stream of nitrogen gas and incubated for 15 min at room temperature to get rid of remaining solvents. 1.5 μL of SensorPrep B1 solution were applied to the sensor and immediately covered with 50 μL of the buffer (150 mM Na-gluconate, 30 mM Hepes pH 7.2/NMG, 10 mM MgCl2, 12.5 mM NaPi pH7.2, freshly added 0.2 mM DTT). Incubate the sensor for 15-60 min at 4°C. An aliquot (10 μL) of inner mitochondrial membrane suspension (stored at - 80°C) were rapidly thawed, diluted with 190 μL of the sensor preparation buffer used above and sonicated in a 1.5 ml Eppendorff tube by applying 5 bursts with an amplitude of 30 % and a cycle time ratio of 0.5 (ultrasonic processor UP 50 H, Dr. Hielscher GmbH, Germany, equipped with MS 1 tip). 5 μL of the membrane suspension was applied onto the sensor surface, representing 4.3 μg total protein

content per sensor. The sensors were centrifuged 45 min at 2500 x *g*, incubated at 4°C for 3-5h and frozen at -80°C. The biosensors were thawed immediately before the experiment and measured with the SURFE2R Workstation 50 or 500 devices. The CII-CIII activity was studied by rapid exchange of a "non-activating" solution for an "activating" solution containing the 3 μM oxidized cytochrome c. 2 s of non-activating buffer was followed by 1 s of activating buffer and then again 1 s non-activating buffer. Afterwards the sensor was rinsed 3 times with 1 mL non-activating buffer. A further activation with cytochrome c was performed after an incubation time of ~11 min. Both buffers contained 1 mM succinate and 350 mg/L BSA to enhance the solubility of the test compounds. For the activity of CII-III, the peak current (current amplitude) was evaluated. The performed measurements of CII-III activities consisted of two parts. First the activity of CII-III was recorded in the absence of compound (instead 0.01% DMSO) for about 50 min to obtain a constant CII-CIII activity (constant peak current amplitudes). Afterwards, a test compound (~4 μM) was supplied to the non-activating and the activating solutions and the CII-III activity was recorded for further ~50 min (110 min in total). For each sensor, all peak currents were normalized to the mean of the activities after 24, 36 and 48 min. For each test compound at least two different biosensors were assayed for -110 min in total, indicated by n (t 0-110) = 2. For those test compounds, which activated CII-III by more than 30% at time 80 to 110 min, the CII-CIII activity was recorded for additional ~50 min (up to 160 min in total).

Results of various compounds used in the invention are reported in Table 4 below.

Table 4

| *Compound* | *Complex II-III activity [%]* |
|---|---|
| ***Compound IA*** | ***102*** |
| ***Compound IC*** | ***97*** |
| ***Compound IE*** | ***110*** |
| ***Compound IF*** | -- |

Functional calory restriction, which has been efficiently shown through studies using SIRT activators, can switch metabolism from glucose to increased use of lipids as major energy source (Lomb et al. Biochim Biophy Acta. 2010; 1804(8):1652-7). Lipids are normally broken down during mitochondrial beta oxidation of fatty acids (FA). Since the electron equivalents of lipid peroxidation are thought to be preferentially fed into the mitochondrial respiratory chain at the level of Complex II (Bruss et al. Am. J. Physiol. Edocrinol. Metab. 2010; 298:E108-E116), it is of importance to assess that the function of this complex is not altered by the treatment.

*E: Microarray Assay and pathway analysis*

Since hardly any reports on the effects of short chain quinones on gene expression patterns *in vivo* are available, we performed microarray analysis of cardiac tissue derived from mice after a long term treatment with Compound IA.

*Experimental Procedure:* Male C57Bl/6 mice were held under standard laboratory conditions (12 hours light per day, 22±2 °C, 40-60% humidity) with food and water available ad libitum. At the age of nine weeks, Compound IA (200 mg/kg, formulated in standard rodent chow) was administered p.o. for a period of three weeks. Vials containing 8 g food mash were administered to single-caged animals at the start of the dark cycle while allowing access to supplementary food *ad libitum.* After the treatment period, animals were sacrificed and the left heart ventricle was immediately excised and lysed for RNA extraction. RNA was purified following standard protocols. Double-stranded cDNA was synthesized from 1.8 μg RNA via single-stranded cDNA reverse transcription using a SuperScript II polymerase and T7-$(N)_6$ primers and subsequent double-strand cDNA synthesis using DNA polymerase 1. Following overnight *in vitro* transcription to generate cRNA, single-stranded cDNA was synthesized by reverse transcription using SuperScript II, random primers and dUTP and thereafter cleaned from RNA templates. Samples were hybridized to the Affymetrix GeneChip® Mouse Exon 1.0 ST Arrays covering over one milllion exon clusters (Affymetrix, Santa Clara, CA, USA) according to instructions by Affymetrix. Analysis of data (principal component analysis; PCA and changes in individual gene expression) was performed using two programs: Partek gene analysis software (Partek Incorporated, St. Louis, MO, USA) and Aligent Genespring GX 10 (Agilent Technologies, Santa Clara, CA, USA). Gene score resampling including all genes were performed using ermineJ (Lee et al, BMC Bioinformatics 6:269, 2005). For functional analysis including only genes that were differently expressed DAVID (Huang DW et al, Nature Protoc. 4(1):44-57, 2009; Dennis G et al, Genome Biol. 4(5):P3, 2003) was used. Gene set enrichment analysis was performed using Ariadne Pathway Studio 6 (Ariadne Genomics, Rockville, MD, USA).

*Results:* After 3-week treatment with Compound IA, more than 650 genes were differently expressed. Multiple forms of analysis, incl. gene score resampling, clearly indicated that the major metabolic parameter in cardiac tissue most affected by Compound IA treatment is fatty acid metabolism.

*F: Effect of cpds on food intake and/or body weight*

Although short chain quinone compounds such as Compound IA are described to have multiple protective effects on mitochondrial function, no information is currently published with regards to changes to metabolism *in vivo.* Due to the results of the microarray analysis, we assessed possible effects of compounds on body weight and food intake.

*Experimental Procedure:* Eight-week-old male C57BL/6J mice were purchased from Janvier (France). After one week acclimatization period in the facility (12 hours light per day, 22±2 °C, 60% humidity), the animals were single-housed in cages with enriched environment (i.e. running wheels) and received a daily dose of 200 mg/kg Compound IA in the food as described above for microarray analysis. Portions which amounted to 75% of the daily calorie intake were supplemented with artificial sweetener for taste preference. Individual portions were stored at -20 °C and administered daily prior to the beginning of dark period. The portions for control animals were prepared identically with the exception of omitted Compound IA. Additionally, mice had access to ad libitum food. Body weight of animals and food intake from ad libitum pellets were measured three times a week over a period of four weeks. Body weight of the animals as well as cumulative food intake relative to body weight was used as endpoints.

*G. Cellular viability of RGC-like cells*

Mitochondrial impairment directly influences cellular survival. Especially cells and tissues that display high level of energy consumption, such as neurons are especially vulnerable. Consequently, these cells and tissues are likely to be the most responsive to a strategy that targets mitochondrial function. We addressed this possibility by assessing the viability of the retinal ganglion cell line RGC-5, challenged with the mitochondrial complex I inhibitor rotenone, in the absence or presence of compounds.

*Experimental procedure:* RGC-5 cells were cultured under ambient conditions (37 °C, 5% CO2, 90% humidity) in DMEM (1g/l glucose; 10% fetal bovine serum (FBS), Penicillin-Streptomycin-Glutamine (PSG)). RGC-5 cells will be seeded in DMEM (1g/l glucose; 2% FBS) into BD Falcon™ cell culture plates. Before rotenone- induced complex I inhibition, the cells were pre-treated with Compound IA or vehicle. For Compound IA co-treatment with rotenone, new compound was added together with rotenone-containing cell culture media. After the rotenone exposure, the media was exchanged and replaced by Hanks' BSS for the 1 day post-incubation. The post-treatment media contained Compound IA or vehicle only. As measure of cell viability, cellular ATP content was analyzed using luminescence from ATP-dependent enzymatic oxidation of luciferin by luciferase. RGC-5 cells were be washed with PBS and lysed in lysis buffer (4 mM EDTA, 0.2% Triton X-100) at room temperature. In 96-well plates, ATP measurement buffer (25 mM HEPES pH 7.25, 300 μM D-luciferin, 5 μg/ml firefly luciferase, 75 μM DTT, 6.25 mM MgCl2, 625 μM EDTA and 1 mg/ml BSA) was combined with lysate to start the reaction. Luminescence was quantified immediately using a multimode plate reader (Tecan M1000, Tecan iControl 1.6 software; Tecan Austria GmbH, Grödig, Austria). Effects on cell viability were defined as the percentage of compound-mediated change in cell viability relative to rotenone-induced reduction in cell viability. Data are presented as mean ± SEM. Paired Student's t-test was used to compare non-normalized data. Statistical significance was set at $p \leq 0.05$(*), $p \leq 0.01$(**) and $p \leq 0.001$(***).

*Results:* In this system, rotenone caused a reduction in cell viability by 59% in DMSO treated RGC-5 cells, while Compound IA significantly rescued viability of RGC-5 cells in a dose dependent manner at concentrations of ≥ 10 nM (see Figure 1). Compound IA treatment including a 1 day pre-incubation mediated a significant rescue in cell viability at 100 nM and 1000 nM, whereas a treatment including a 2 days Compound IA pre-incubation mediated a significant rescue of cell viability already at 10 nM. Specifically, pre-incubation with Compound IA for 1 day (Figure 1, white bars) increased RGC-5 viability by up to 26% (at 1 μM), while concentrations of 1 and 10 nM showed a slight, but not significant, protective effect. In comparison to a 1 day pre-treatment (Figure 1, white bars), protection of cellular viability was significantly improved by a 2 day pre-treatment (Figure 1, black bars). At concentrations of 10 nM, 100 nM and 1000 nM Compound IA significantly increased cellular viability by 7%, 35% and 58%, respectively.

Cellular survival of neuronal cells is thought to be influenced by neurotrophic factors such as NGF. Therefore, compounds that can induce the expression of neurotrophic factors such as propentophilline are considered to be potentially neuroprotective. However, when Compound IA was compared to propentophylline (Figure 2) with regards to a protective role in the cell culture system of mitochondrial dysfunction described above, propentophilline (100 μM) demonstrated no significant protective activity ($p>0.05$) while the protection of cellular survival by Compound IA (1 μM) was highly significant ($p=0.0005$).

| Fig.1 | 1 day-preincubation with Compound IA | | | | 2 day-preincubation with Compound IA | | | |
|---|---|---|---|---|---|---|---|---|
| | 1 nM | 10 nM | 100 nM | 1000 nM | 1 nM | 10 nM | 100 nM | 1000 nM |
| mean | 4.0 | 4.8 | 12.6 | 25.9 | 1.0 | 7.3 | 34.9 | 58.4 |

(continued)

| Fig.1 | 1 day-preincubation with Compound IA | | | | 2 day-preincubation with Compound IA | | | |
|---|---|---|---|---|---|---|---|---|
| StDev | 6.3 | 4.7 | 3.0 | 5.1 | 4.8 | 5.3 | 6.6 | 6.1 |
| **Fig. 2** | **1 day-preincubation with Compound IA (IA) or propentophylline (PP)** | | | | | | | |
| | IA | PP | | | | | | |
| mean | 22.0 | 2.2 | | | | | | |
| StDev | 1.5 | 2.0 | | | | | | |

*H. Mouse model for diseases associated with mitochondrial impairment in eye diseases*

This mouse model is used to assess the therapeutic potential of compounds in eye disorders characterized by mitochondrial dysfunction such as LHON, DOA, macular degeneration, retinopathy, and complex I deficiency. It is a widely accepted *in vivo* model based on single-eye intravitreal injection of the mitochondrial complex I inhibitor rotenone in the adult mouse. Intravitreal injection of rotenone induces neurotoxicity through mitochondrial complex I dysfunction in the retina which is the main molecular dysfunction associated with. The impact of oral pre-treatment with test compounds for 3 weeks is histologically assessed on retinal integrity at different time points after rotenone injection. Histological analysis includes quantitative measurement of the retinal thickness (whole retina and individual layers), RGC apoptosis and cell death, oxidative stress, glutamine synthetase expression, and gliosis. The non-injected eye is used as a within subject, i.e. intraindividual, control.

*Experimental procedure (I): Histopathological analyses of retinal degeneration*

Male wild-type C57BL/6J mice (7 weeks old, about 25 g body weight) are individually housed in Makrolon type II cages with standard bedding and free access to food and water under a regular 12-hours light-dark cycle (8 a.m. to 8 p.m.). Mice are treated once daily for 3 weeks with a dose range of the test compound (20 to 2000 mg/kg of body weight in CMC 0.5%) or with vehicle (CMC 0.5%) administered in diet (mixed 1:1 with autoclaved food powder supplemented with 10% sucrose; 5.5 g/day) or by gavage (p.o.; 10 ml/kg of body weight/day) prior to a single-eye intravitreous injection of rotenone (15 mM in DMSO, 1 μl) or DMSO vehicle (1 μl). Mice are anesthetized (by inhalation of 1.5% to 2% isoflurane) and rotenone (or DMSO vehicle) is injected into the vitreous chamber of the right eye using a 10-μl Hamilton syringe adapted with a small needle. The needle tip is inserted into the superior hemisphere of the eye, at the level of the pars plana and with a 45° angle through the sclera. The injection is performed over a period of 2 min and the needle is kept in place for an additional 3 min, after which it is gently removed. Sterile surgical glue (Histoacryl, BRAUN/Aesculap AG, Germany) is used to seal the site of injection and a sterile antimicrobial ophtalmic ointment (Neosporin, GlaxoSmithKline, UK) is applied to the injected eye to avoid any infection. The mice are then returned to their home cages where they are continuously exposed to the test compound (or vehicle) until sacrificed for histological analysis (1 and 7 days following rotenone injection). Briefly, mice are anesthetized by i.p. injection of ketamin/xylazin (2/1, 200μl) and subsequently perfused intracardiacally with a slolution of 4% paraformaldehyde in phosphate buffer (PB 0.1 M) for eye fixation. The eye balls are removed, retinas are dissected, fixed for an additional 2 h at 4°C, incubated in PB 0.1 M and 30% sucrose overnight, frozen in optimal cutting temperature compound (O.C.T., Tissue-Tek, Miles Laboratories, Elkhart, IN, USA), sliced at 14 μm, and collected onto glass slides for immunostaining. Immunostaining is performed accordingly to conventional method using multiple primary antibodies. Fluorescent-conjugated secondary antibodies and DAPI are used for visualization under a fluorescence microscope. All procedures are performed in accordance with the Swiss regulation and under a license approved by the "Kantonales Veterinäramt Basel-Land".

*Results:* In a first study, pre-treatment of adult C57/B6 mice with Compound IA (400 and 2000mg/kg/day) for 3 weeks showed strong protective effects against toxicity of intravitreal injection of the complex I inhibitor rotenone, regarding multiple LHON related endpoints such as cell death of retinal ganglion cells (RGC), reduced retinal thickness, gliosis and expression of glutamine synthetase. The results are shown in Table 5 below.

Table 5

| Treatment/Endpoint | **RGC number** | **Retinal thickness** | **Gliosis** | **Glutamine-synthetase** |
|---|---|---|---|---|
| **Sham (DMSO) - injected** | 100 ± 12.2 (n=3) | 100 ± 4.1 (n=6) | 100 ± 9.7 (n=6) | 100 ± 0.5 (n=3) |
| **Vehicle + rotenone** | 73.5 ± 2.9 (n=3) | 61.5 ± 3.9 (n=4) | 241.4 ± 33.7 (n=4) | 89.4 ± 3.3 (n=6) |

(continued)

| Treatment/Endpoint | RGC number | Retinal thickness | Gliosis | Glutamine-synthetase |
|---|---|---|---|---|
| **IA 400 + rotenone** | 94.6 ± 3.2 ** (n=3) | 84.3 ± 2.9 ** (n=3) | 112.7 ± 11.9 * (n=3) | 98.5 ± 1.9 (n=5) |
| **IA 2000 + rotenone** | 101.3 ± 11.3 (n=3) | 76.9 ± 10.1 (n=4) | 134.7 ± 10.7* (n=3) | 99 ± 2.9 * (n=6) |

In Table 5, the histological data are expressed as % of sham (DMSO) injected control ± SEM. The RGC number is based on RGC cells / $mm^2$ on 1 day post-injection; the retinal thickness (μm) is based on a 7 days post-injection; gliosis is based on relative fluorescence units (RFU) of GFAP-specific immunostaining on 7 days post-injection; glutamine-synthetase (GS) is based on relative fluorescence units (RFU) of GS-specific immunostaining on 1 day post-injection. IA 400 means a pretreatment with Compound IA at 400 mg/kg, similarly IA 2000 means a pretreatment with Compound IA at 2000 mg/kg. Statistical significance relative to vehicle + rotenone group is $p \leq 0.05$ (*), $p \leq 0.01$ (**). n is the number of retinas per group.

These results were replicated in a second *in vivo* study with the inclusion of a lower concentration of Compound IA (namely 200mg/kg/day), using the same model system. A modified, less invasive injection method was used to further minimize damage caused by the injection itself. As a consequence no significant reduction in RGC number was detected 24h after injection of rotenone and consequently, no protective effect could be demonstrated at this time point. Nevertheless, 7 days after the rotenone challenge, RGC numbers were significantly reduced (< 35 % residual cells) compared to the vehicle-injected control (Table 2). Under these conditions Compound IA showed a significant protection of RGC survival at doses of 400 and 2000 mg/kg and a trend (p = 0.102) towards protection at a dose of 200 mg/kg. Compound IA also significantly protected against decreased retinal thickness 7 days after rotenone-injection (Table 2). While rotenone, on average, reduced retinal thickness from about 300 μm (sham-injected) to 220 μm (rotenone-injected), Compound IA pre-treatment prevented this reduction to different extent depending on the concentration. Animals pre-treated with Compound IA with 200 mg/kg showed protection of retinal thickness (272 μm), although this effect did not reach significance (p = 0.1347). At 400 mg/kg Compound IA, a clear significant protection was observed (269 μm; p = 0.03), whereas at 2000 mg/kg a strong protective trend was detected (258 μm; p = 0.06). Furthermore, in response to rotenone, strong fiber-associated GFAP-specific signal, indicative of gliosis, was evident after 7 days, which was largely reduced by Compound IA. The obtained data are summarized in Table 6 below.

**Table 6**

| Treatment | RGC number | Retinal thickness | Gliosis |
|---|---|---|---|
| **S+V** | 100 ± 7.33 (n=8) | 100 ± 5.2 (n=6) | 100 ±9.7 (n=7) |
| **R+V** | 34.79 ± 9 (n=3) | 73.74 ± 7.92 (n=3) | 178.14 ± 21.6 (n=3) |
| **R + IA 200** | 62.53 ± 9.13 (n=7) | 91.17 ± 5.72 (n=8) | 124.46 ± 7.37 * (n=7) |
| **R + IA 400** | 63.68 ± 5.98 * (n=6) | 89.93 ± 2.16 * (n=6) | 125.04 ±9.12 * (n=6) |
| **R + IA 2000** | 64.44 ± 4.56 * (n=8) | 86.58 ± 2.51 (n=8) | 129.86 ± 7.32 * (n=7) |

In Table 6, the histological data on 7 days post-injection is expressed as % of sham (DMSO) injected control ± SEM. The RGC number is based on RGC cells / $mm^2$; the retinal thickness is measured in μm and gliosis is based on relative fluorescence units (RFU) of GFAP-specific immunostaining. "S" within Table 6 means sham-injected (DMSO); "V" means vehicle-treated; "R" means rotenone-injected. The term IA 200 defines a treatment with Compound IA at a concentration of 200 mg/kg; similiarily, IA 400 and IA 2000: define a treatment with Compound IA at 400 mg/kg and at 2000 mg/kg, respectively. Statistical significance relative to vehicle + rotenone group is expressed as $p \leq 0.05$ (*). n designates the number of retinas per group.

<u>*Experimental procedure (II):*</u> Optomotor testing

Since RGC loss is associated with visual acuity impairment, we tested whether the neuroprotective effect of the test compound IA also correlated with recovery of visual function. For this purpose, visual acuity was detected by quantifying the optomotor response of animals subjected to the test system described above. The optomotor response was measured as previously described (see Jellali A., Meziane H., Ouagazzal A-M., et al. Vis. Res., 2005). Mice were placed on a central platform surrounded by a motorized drum with black and white vertical stripes that can rotate in both, clockwise and counterclockwise, direction at different velocities (which can be adjusted to evoke the optimal optokinetic

response), and under constant light levels. Mice were allowed to habituate for 5 min to the experimental conditions before stripes were rotated alternately clockwise and counterclockwise, for 2 min in each direction and with an interval of 30 s between the two rotations. The mice were recorded on a computer using a digital video camera mounted above the apparatus to subsequently score the number of head movements by manual counting. Head movements were scored only when the angular speed of the head turn corresponds to that of the drum rotation. After aquisition and encoding by an investigator, a second, blinded investigator conducted all assessments. Codes were broken upon completion of data acquisition by a third investigator. Each mouse was tested at different time points and the apparatus will be cleaned between mice. Data will be presented as mean ± SEM. Paired Student's t-test and analysis of variance (ANOVA) will be used to compare non-normalized data. Statistical significance will be set at $p \leq 0.05$(*), $p \leq 0.01$(**) and $p \leq 0.001$ (***).

**Table 7: Effect of idebenone pre-treatment on visual acuity in an *in vivo* mouse model for LHON.**

| | Number of head movements | |
|---|---|---|
| Time post-injection (days) | Vehicle | Idebenone (2000 mg/kg) |
| -7 | 7.6 ± 1.2 (n=5) | 9.7 ± 0.7 (n=7) |
| 1 | 0 ± 0 (n=5) | 0 ± 0 (n=7) |
| 7 | 0 ± 0 (n=5) | 0.5 ± 0.5 (n=7) |
| 14 | 0 ± 0 (n=5) | 1.8 ± 0.8 (n=7) |
| 21 | 0 ± 0 (n=5) | 2.8 ± 1.4 (n=7) |
| 28 | 0 ± 0 (n=5) | 2.8 ± 1.4 (n=7) |
| 35 | 0.4 ± 0.4 (n=5) | 3.5 ± 1.5 (n=7) |
| 56 | 0 ± 0 (n=3) | 6 ± 2.1 (n=4) |

Quantification of mouse visual acuity based on the optomotor response in mice pre-treated for 3 weeks with vehicle and idebenone 2000 mg/kg in diet. Results are expressed as mean of head movements/2 min ± SEM; n = number of mice per group.

Quantification of the optomotor response 5 weeks after injection of 1 and 5 mM rotenone. Mice were treated for 3 weeks with vehicle and idebenone 2000 mg/kg in diet prior rotenone injection. The optomotor response was examined in both clockwise (a) and counterclockwise (b) directions of motion, clockwise and counterclockwise tracking head movements reflecting visual acuity of the rotenone-injected (left) and non-injected (right) eye, respectively. Number of clockwise head movements/ 2 min: vehicle + rotenone 1 mM = 0.5 ± 0.5 (n = 2), vehicle + rotenone 5 mM = 0.4 ± 0.4 (n = 5), idebenone 2000 mg/kg + rotenone 1 mM = 9 ± 2 (n = 2), idebenone 2000 mg/kg + rotenone 5 mM = 3.5 ± 1.5 (n = 7). Number of counterclockwise head movements/ 2 min: vehicle + rotenone 1 mM = 12 ± 0 (n = 2), vehicle + rotenone 5 mM = 13.6 ± 1 (n = 5), idebenone 2000 mg/kg + rotenone 1 mM = 12.5 ± 4.5 (n = 2), idebenone 2000 mg/kg + rotenone 5 mM = 10.5 ± 0.6 (n = 6). Results are expressed as mean of head movements/2 min ± SEM; n = number of mice per group; id2000 = idebenone 2000 mg/kg body weight.

*Results:*

After intravitreal injection of rotenone, animals that were sham-treated did loose visual acuity (evidenced by a total loss of clockwise head movements). Animals orally pre-treated with test compound IA however, regained visual acuity over a period of 56 days (Table 7, Figure 3 and 4).

*I. Pharmacokinetic analysis of the test compound and related metabolites in plasma and eye fluids*

Pharmacokinetic analysis of test compounds after oral administration aims to determine its bioavailability and clearance in the plasma and in the eye fluids (aqueous and vitreous humor). The test compounds are quantified in the plasma and in the aqueous and vitreous humor after acute and repeated treatment (with different routes of administration). The time-dependent concentration is then determined and pharmacokinetic parameters ($C_{max}$, $AUC_{0-t}$, $t_{max}$) are calculated.

*Experimental procedure:* Male wild-type C57BL/6J mice (7 weeks old, about 25 g body weight) are individually housed in Makrolon type II cages with standard bedding and free access to food and water under a regular 12-hours light-dark cycle (8 a.m. to 8 p.m.). Mice receive single or daily repeated oral doses of the test compounds (ranging from 20 to 2000 mg/kg of body weight) or the vehicle administered though the diet or by gavage at the end of the light phase.

The treatment can be followed by a washout period. Samples for pharmacokinetic analysis are taken at different time intervals following compound administration. Mice are anesthetized by 5% isofluran inhalation in an induction chamber and sacrificed by cervical dislocation. Blood is collected from the head cut for plasma extraction. Eye balls are dissected out to collect aqueous and vitreous humor. Samples are snap frozen in liquid nitrogen and stored (-80°C) until analyzed by LC-MS/MS and accordingly to standardized procedures. All mouse procedures are performed in accordance with the Swiss regulation and under a license approved by the "Kantonales Veterinäramt Basel-Land".

The pharmacokinetic profile of idebenone (compound IA) was assessed, following a single oral gavage dose to male mice. Following a single oral dose of 400 mg/kg idebenone concentrations in plasma, and vitreous and aqueous humors, generally reached a maximum at the first sampling time (15 min post-dose), suggesting rapid absorption and rapid uptake of idebenone into the eye (Table 8). Thereafter, concentrations of idebenone in plasma, and vitreous and aqueous humors generally declined over a 6 h post-dose period, suggesting a lack of sequestration of idebenone in the eye. Furthermore, the ratio (vitreous humor /plasma and aqueous humor/plasma) appeared to be independent of the time post dose, consistent with a lack of accumulation of idebenone in the eye. The overall extent of exposure to idebenone in the vitreous humor, as measured by $AUC_{0-6h}$, represented about 2 % of that observed in plasma (vitreous/plasma ratio; Table 8). Similarly, the extent of exposure to idebenone in the aqueous humor represented about 3 % of that observed in plasma (aqueous/plasma ratio; Table 8).

The apparent terminal half-life ($t_{1/2}$) of idebenone in plasma was 1.70 h, respectively (Table 8). (The apparent terminal half-lifes $t_{1/2}$ of idebenone could not be estimated in vitreous humor and in aqueous humor, due to limited data).

**Table 8 Pharmacokinetic parameters of Compound IA, in plasma, vitreous humor and aqueous humor following single oral administration at 400 mg/kg to male mice.**

**Plasma**

| Analyte | $C_{max}$ (ng/mL) | $t_{max}$ (h) | $AUC_{0-6h}$ (ng.h/mL) | $AUC_{0-\infty}$ (ng.h/mL) | $\lambda_z$ (/h) | $t_{1/2}$ (h) |
|---|---|---|---|---|---|---|
| **IA** | 1070 | 0.25 | 1857 | 1963 | 0.409 | 1.70 |

**Vitreous Humor**

| Analyte | $C_{max}$ | $t_{max}$ | $AUC_{0-6h}$ | $AUC_{0-\infty}$ | $\lambda_z$ | $t_{1/2}$ | Vitreous/plasma | |
|---|---|---|---|---|---|---|---|---|
| | (ng/mL) | (h) | (ng.h/mL) | (ng.h/mL) | (/h) | (h) | $C_{max}$ | $AUC_{0-6h}$ |
| **IA** | 37.9 | 0.25 | 38.1 | NC | NC | NC | 0.0354 | 0.0205 |

**Aqueous Humor**

| Analyte | $C_{max}$ | $t_{max}$ | $AUC_{0-6h}$ | $AUC_{0-\infty}$ | $\lambda_z$ | $t_{1/2}$ | Aqueous /plasma | |
|---|---|---|---|---|---|---|---|---|
| | (ng/mL) | (h) | (ng.h/mL) | (ng.h/mL) | (/h) | (h) | $C_{max}$ | $AUC_{0-6h}$ |
| **IA** | 86.2 | 0.25 | 62.6 | NC | NC | NC | 0.0806 | 0.0337 |

NC: Not calculated; a terminal monoexponential phase could not be unambiguously identified

*Results:*

In summary, following oral dietary or gavage administration of Compound IA to the mouse there was a rapid absorbtion of idebenone from the GI tract.

Surprisingly, a rapid up-take of idebenone into the eye, i.e. the vitreous and aqueous humors of the mouse (maximum < 15 min post-dose) was observed. To our knowledge, there is no precedence in the literature of any study referring to examination of eye levels of compound IA. In particular, there is no precedence of any measurement of idebenone eye levels after simple oral administration (by gavage or in diet).

Hence, we surprisingly found that a simple oral dosing (by gavage or in diet) results in sufficient levels of Compound IA in the eye to exert its beneficial effects on mitochondrial diseases like LHON, autosomal dominant optic atrophy (DOA), macular degeneration, glaucoma, retinopathy, cataract, optic disc drusen (ODD), mitochondrial myopathy, encephalomyopathy, lactic acidosis, stroke-like symptoms (MELAS), myoclonic epilepsy with ragged red fibers (MERRF), myoneurogenic gastrointestinal encephalomyopathy (MNGIE), Kearns-Sayre syndrome, CoQ10 deficiency, or mitochondrial complex deficiencies (1-5, CPEO) as all these diseases are associated with an ophthalmological phenotype.

Corresponding concentrations of Compound IA in vitreous and aqueous humor increased approximately dose proportionately. Thus, as an important feature with regards to therapeutic developability, Compound IA (IA) exhibits linear kinetics of absorbtion into the eye fluids, since the ratio (vitreous humor /plasma and aqueous humor/plasma) is

independent of the dose used. Furthermore, there was no accumulation of Compound IA in the eye, since the ratio (vitreous humor /plasma and aqueous humor/plasma) is independent of dosing duration or time post-dosing. There was also no sequestration of Compound IA found in the eye.

## Claims

1. A compound represented by the general formula (I)

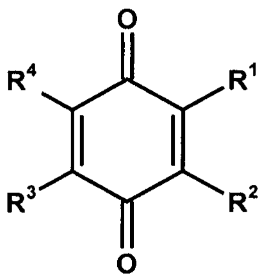

(I)

and the enantiomers, tautomers, solvates or pharmaceutically acceptable salts thereof, wherein
$R^1$ is a substituent represented by formula (II):

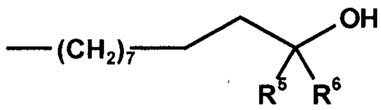

(II)

wherein

$R^5$ and $R^6$ are both hydrogen atoms, or
$R^5$ is a hydrogen atom and $R^6$ is an ethyl group, or
$R^5$ and $R^6$ are both methyl groups,

$R^2$ is a methyl group,
$R^3$ is a methoxy group, and
$R^4$ is a methoxy group;
for use in the treatment of a mitochondrial disease, wherein the mitochondrial disease is selected from the group consisting of Leber's hereditary optic neuropathy (LHON), autosomal dominant optic atrophy (DOA), macular degeneration, glaucoma, retinopathy, cataract and optic disc drusen (ODD).

2. The compound for use according to claim 1, wherein $R^5$ and $R^6$ are both hydrogen atoms.

3. The compound for use according to claim 2 wherein the compound is orally administered.

4. The compound for use according to claim 3, wherein the mitochondrial disease is selected from the group consisting of Leber's hereditary optic neuropathy (LHON), autosomal dominant optic atrophy (DOA), macular degeneration and glaucoma.

5. Use of a compound according to general formula (I)

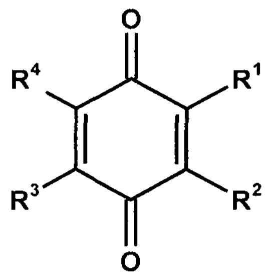

(I)

wherein $R^1$ to $R^4$ are defined as in claim 1 for the preparation of a medicament in the treatment of a mitochondrial disease, wherein the mitochondrial disease is selected from the group consisting of Leber's hereditary optic neuropathy (LHON), autosomal dominant optic atrophy (DOA), macular degeneration, glaucoma, retinopathy, cataract and optic disc drusen (ODD).

**Patentansprüche**

1. Verbindung, dargestellt durch die allgemeine Formel (I)

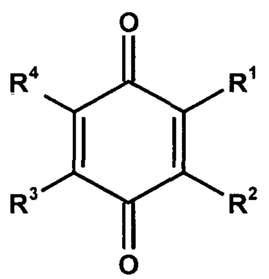

(I)

und die Enantiomere, Tautomere, Solvate oder pharmazeutisch verträglichen Salze davon, wobei
$R^1$ ein Substituent ist, dargestellt durch die Formel (II):

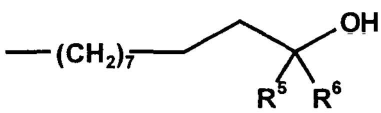

(II)

wobei

$R^5$ und $R^6$ beide Wasserstoffatome sind, oder
$R^5$ ein Wasserstoffatom und $R^6$ eine Ethylgruppe ist, oder
$R^5$ und $R^6$ beide Methylgruppen sind,

$R^2$ eine Methylgruppe ist,
$R^3$ eine Methoxygruppe ist und
$R^4$ eine Methoxygruppe ist;
zur Verwendung in der Behandlung einer mitochondrialen Krankheit, wobei die mitochondriale Krankheit ausgewählt ist aus der Gruppe bestehend aus Leberscher hereditärer Optikusneuropathie (LHON), autosomal-dominanter optischer Atrophie (DOA), Makuladegeneration, Glaukom, Retinopathie, Katarakt und Drusenpapille (ODD).

2. Verbindung zur Verwendung gemäß Anspruch 1, wobei $R^5$ und $R^6$ beide Wasserstoffatome sind.

3. Verbindung zur Verwendung gemäß Anspruch 2, wobei die Verbindung oral verabreicht wird.

**4.** Verbindung zur Verwendung gemäß Anspruch 3, wobei die mitochondriale Krankheit ausgewählt ist aus der Gruppe bestehend aus Leberscher hereditärer Optikusneuropathie (LHON), autosomal-dominanter optischer Atrophie (DOA), Makuladegeneration und Glaukom.

**5.** Verwendung einer Verbindung gemäß der allgemeinen Formel (I)

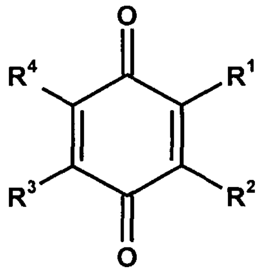

(I)

wobei $R^1$ bis $R^4$ wie in Anspruch 1 definiert sind, für die Herstellung eines Arzneimittels in der Behandlung einer mitochondrialen Krankheit, wobei die mitochondriale Krankheit ausgewählt ist aus der Gruppe bestehend aus Leberscher hereditärer Optikusneuropathie (LHON), autosomal-dominanter optischer Atrophie (DOA), Makuladegeneration, Glaukom, Retinopathie, Katarakt und Drusenpapille (ODD).

**Revendications**

**1.** Composé représenté par la formule générale (I)

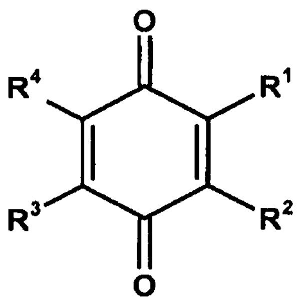

(I)

et les énantiomères, tautomères, solvates, ou sels pharmaceutiquement acceptables de celui-ci,
où
$R^1$ est un substituant représenté par la formule (II) :

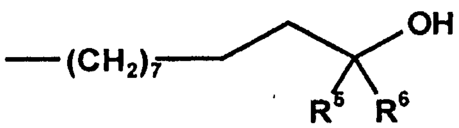

(II)

dans laquelle

$R^5$ et $R^6$ sont tous deux des atomes d'hydrogène, ou
$R^5$ est un atome d'hydrogène et $R^6$ est un groupe éthyle, ou
$R^5$ et $R^6$ sont tous deux des groupes méthyle,
$R^2$ est un groupe méthyle,
$R^3$ est un groupe méthoxy, et
$R^4$ est un groupe méthoxy ;

pour utilisation dans le traitement d'une maladie mitochondriale, dans lequel la maladie mitochondriale est sélectionnée dans le groupe constitué par la neuropathie optique héréditaire de Leber (NOHL), l'atrophie optique autosomique dominante (AOAD), la dégénérescence maculaire, le glaucome, la rétinopathie, la cataracte et les drusen de la papille.

2. Composé pour utilisation selon la revendication 1, dans lequel $R^5$ et $R^6$ sont tous deux des atomes d'hydrogène.

3. Composé pour utilisation selon la revendication 2, lequel composé est administré par voie orale.

4. Composé pour utilisation selon la revendication 3, dans lequel la maladie mitochondriale est sélectionnée dans le groupe constitué par la neuropathie optique héréditaire de Leber (NOHL), l'atrophie optique autosomique dominante (AOAD), la dégénérescence maculaire et le glaucome.

5. Utilisation d'un composé répondant à la formule générale (I)

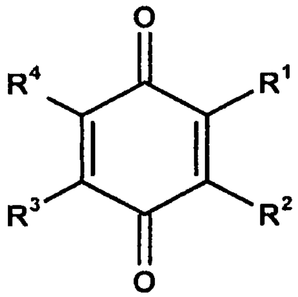

(I)

dans laquelle les $R^1$ à $R^4$ sont définis comme dans la revendication 1 pour la préparation d'un médicament destiné au traitement d'une maladie mitochondriale, la maladie mitochondriale étant sélectionnée dans le groupe constitué par la neuropathie optique héréditaire de Leber (NOHL), l'atrophie optique autosomique dominante (AOAD), la dégénérescence maculaire, le glaucome, la rétinopathie, la cataracte et les drusen de la papille.

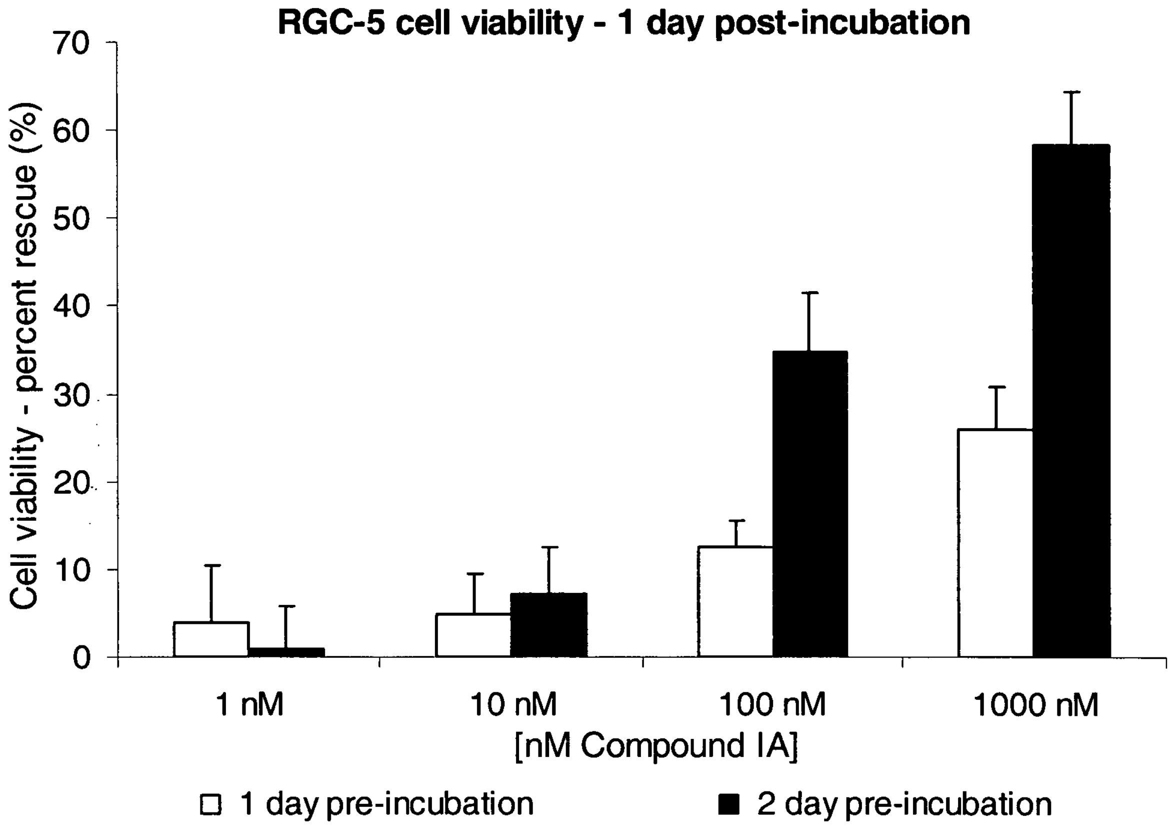


Figure 1: Effect of compound IA on cellular survival of RGC cells in response to mitochondrial impairment by rotenone

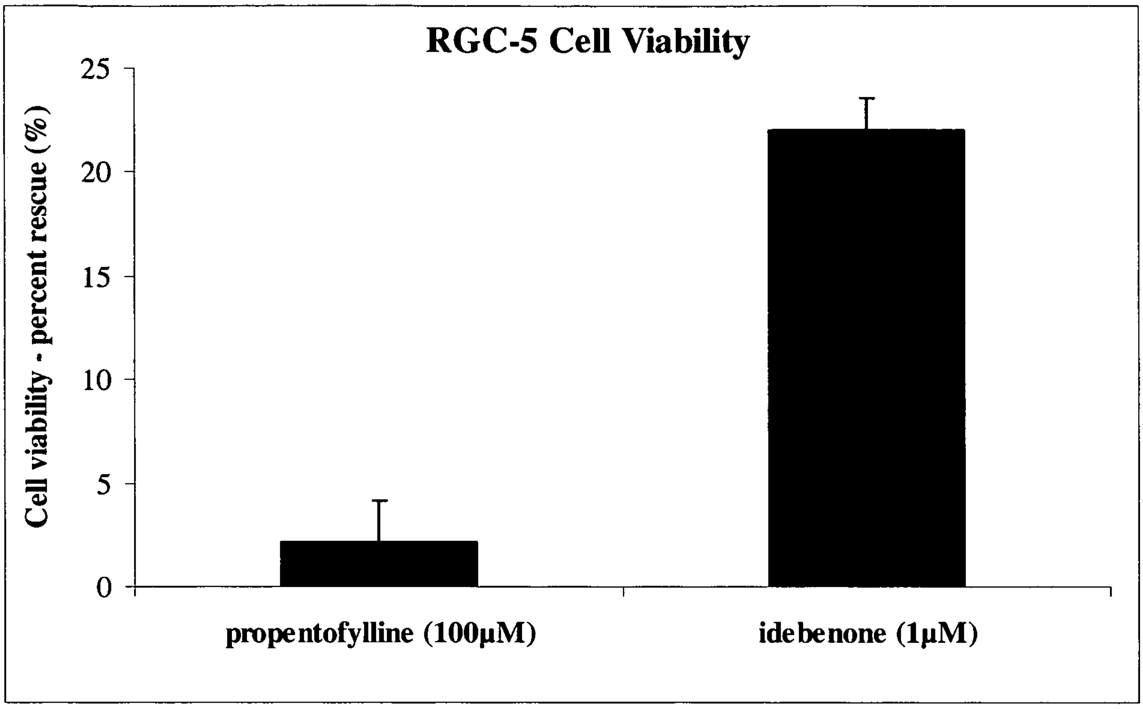


Figure 2: Comparison of compound IA and propentophylline on cellular survival of RGC cells in response to mitochondrial impairment by rotenone. While cellular protection by propentophylline was not significant ($p>0.05$), the effect of compound IA was highly significant ($p=0.0005$)

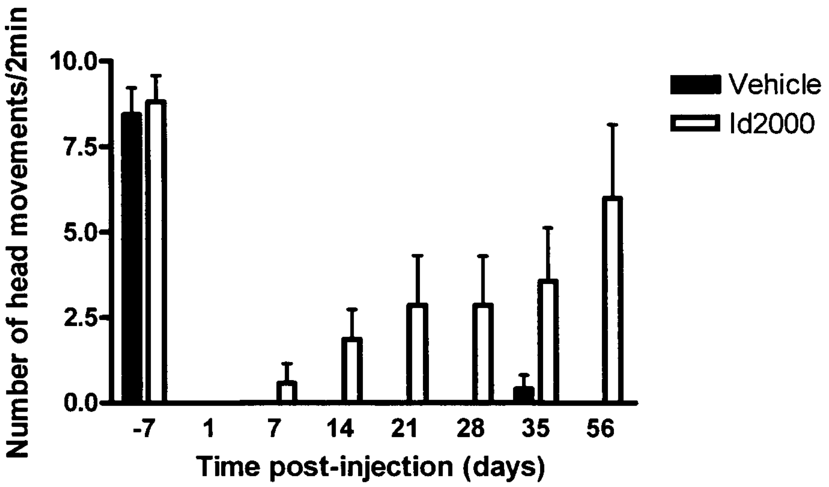


Figure 3: Effect of Compound IA pre-treatment on visual acuity in an *in vivo* mouse model for eye disorders characterized by mitochondrial dysfunction.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006130775 A
- EP 0201196 A
- JP 58077839 A
- WO 2006100017 A
- JP 62003134 B
- JP 58077839 B
- EP 0038674 A
- US 20100130619 A
- US 20100129431 A1
- US 20100215725 A1
- US 20100099775 A1
- WO 2008019769 A
- WO 2000032197 A
- US 4139545 A, Takeda


### Non-patent literature cited in the description


- **CHRYSOSTOMOU V ; TROUNCE IA ; CROWSTON JG.** *Ophthalmic Res.,* 2010, vol. 44 (3), 173-8
- **JOU et al.** *Chang Gung Med J.,* 2009, vol. 32 (4), 370-9
- **YANG et al.** *DNA Repair,* 2008, vol. 7 (7), 1110-20
- **PUDDU et al.** *J Biomed Sci.,* 2009, vol. 16, 112
- **SINGH ; KULAWIEC.** *Methods Mol Biol.,* 2009, vol. 471, 291-303
- **BURCHELL et al.** *Expert Opin Ther Targets,* 2010, vol. 14 (4), 369-85
- **PIECZENIK ; NEUSTADT.** *Exp Mol Pathol.,* 2007, vol. 83 (1), 84-92
- **COMPSTON A.** *Int MS J .,* 2003, vol. 10, 29-31
- **HAFLER DA.** *J. Clin. Invest.,* 2004, vol. 113, 788-794
- **FILIPPINI G et al.** *Lancet,* 2003, vol. 361, 545-552
- **K. OKAMOTO et al.** synthesis, metabolism and in vitro biological activities of 6-(10-hydroxydecyl)-2,3-dimethoxy-5-methyl-1,4-benzoquinone-related compounds. *Chem. Pharm. Bull.,* 1988, vol. 36 ((1)), 178-189
- **D. Y. DUVEAU et al.** prepared and investigated analogues of mitoQ and idebenone to define the structural elements that support oxygen consumption in the mitochondrial respiratory chain. *Bioorg. Med. Chem.,* 2010, vol. 18, 6429-6411
- **OKAMOTO et al.** *Chem. Pharm. Bull.,* 1988, vol. 36, 178
- *Molecular Carcinogenesis,* 2010, vol. 49 (4), 324-336
- *Chemico-Biological Interactions,* 2007, vol. 167 (2), 125-134
- *Tetrahedron,* 1998, vol. 54 (49), 14791-802
- *Phytotherapy Research,* 2000, vol. 14 (7), 510-6
- **TORII H ; YOSHIDA K ; KOBAYASHI T ; TSUKAMOTO T ; TANAYAMA S.** *J Pharmacobiodyn.,* June 1985, vol. 8 (6), 457-67
- **TORII H ; YOSHIDA K ; KOBAYASHI T ; TSUKAMOTO T ; TANAYAMA S.** *J Pharmacobiodyn.,* June 1985, vol. 8 (6), 457-67
- **NAGAI Y ; YOSHIDA K ; NARUMI S ; TANAYAMA S ; NAGAOKA A.** Brain distribution of idebenone and its effect on local cerebral glucose utilization in rats. *Arch Gerontol Geriatr.,* 1989, vol. 8 (3), 257-72
- **STEUER H ; KIM, JH et al.** Functional characterization and comparison of the outer blood-retina barrier and the blood brain barrier. *Invest Ophthalmol Vis Sci.,* March 2005, vol. 46 (3), 1047-53
- **ZHANG X. ; JONES D. ; GONZALEZ-LIMA F.** Mouse model of optic neuropathy caused by mitochondrial complex I dysfunction. *Neurosci. Lett.,* 2002
- **TAKEUCHI, R. ; MURASE, K. ; FURUKAWA, Y. ; FURUKAWA, S. ; HAYASHI, K.** Stimulation of nerve growth factor synthesis/secretion by 1,4-benzoquinone and its derivatives in cultured mouse astroglial cells. *FEBS Lett.,* 1990, vol. 261, 63-66
- **SHINODA et al.** Stimulation of nerve growth factor synthesis/secretion by propentophylline in cultured mouse astroglial cells. *Biochem. Pharmacol.,* 1990, vol. 39, 1813-1816
- **WONG-RILEY M.** Energy Metabolism of the Visual System. *Eye and Brain,* 2010, vol. 2, 99-116
- **LIESA M et al.** *Physiol Rev.,* 2009, vol. 89, 799-845
- **FEHER J ; KOVACS I ; ARTICO M ; CAVALLOTTI C ; PAPALE A ; BALACCO GABRIELI C.** *Neurobiol Aging.,* July 2006, vol. 27 (7), 983-937

- **FEHER J ; PAPALE A ; MANNINO G ; GUALDI L ; BALACCO GABRIELI C.** *Ophthalmologica,* September 2003, vol. 217 (5), 351-7
- **GUO Y ; JOHNSON EC ; CEPURNA WO ; DYCK J ; DOSER T ; MORRISON JC.** *Invest Ophthalmol Vis Sci.,* 04 November 2010
- **JU WK ; KIM KY ; DUONG-POLK KX ; LINDSEY JD ; ELLISMAN MH ; WEINREB RN.** Increased optic atrophy type 1 expression protects retinal ganglion cells in a mouse model of glaucoma. *Mol Vis.,* 15 July 2010, vol. 16, 1331-42
- **JARRETT SG ; LEWIN AS ; BOULTON ME.** *Ophthalmic Res.,* 2010, vol. 44 (3), 179-90
- **LEE S ; VAN BERGEN NJ ; KONG GY ; CHRYSOSTOMOU V ; WAUGH HS ; O'NEILL EC ; CROWSTON JG ; TROUNCE IA.** *Exp Eye Res.,* 04 August 2010
- **TSO MO.** *Ophthalmology,* October 1981, vol. 88 (10), 1066-80
- **JONES MM ; MANWARING N ; WANG JJ ; ROCHTCHINA E ; MITCHELL P ; SUE CM.** *Arch Ophthalmol.,* September 2007, vol. 125 (9), 1235-40
- **FRIEDERICH M ; HANSELL P ; PALM F.** *Curr Diabetes Rev.,* May 2009, vol. 5 (2), 120-44
- **BUGGER H ; ABEL ED.** *Clin Sci,* February 2008, vol. 114 (3), 195-210
- **BOUMEZBEUR F ; MASON GF ; DE GRAAF RA ; BEHAR KL ; CLINE GW ; SHULMAN GI ; ROTHMAN DL ; PETERSEN KF.** *J Cereb Blood Flow Metab.,* 30 September 2009
- **ISHIKAWA K ; KOSHIKAWA N ; TAKENAGA K ; NAKADA K.** *Hayashi J Mitochondrion,* 2008, vol. 8 (4), 339-44
- **ISHIKAWA S ; MIMA T ; AOKI C ; YOSHIO-HOSHINO N ; ADACHI Y ; IMAGAWA T ; MORI M ; TOMIITA M ; IWATA N ; MURATA T.** *Ann Rheum Dis.,* 2009, vol. 68 (2), 264-72
- **VICTOR VM ; ESPULGUES JV ; HERNÁNDEZ-MIJARES A ; ROCHA M.** *Infect Disord Drug Targets,* 2009, vol. 9 (4), 376-89
- **J.J. BEDNARSKI ; R.E. WARNER ; T. RAO ; F. LEONETTI ; R. YUNG ; B.C. RICHARDSON ; K.J. JOHNSON ; J.A. ELLMAN ; A.W. OPIPARI JR. ; G.D. GLICK.** *Arthritis & Rheumatism,* 2003, vol. 48, 757
- **ARNOULT D ; CARNEIRO L ; TATTOLI I ; GIRARDIN SE.** *Semin Immunol.,* 2009, vol. 21 (4), 223-32
- **CHODACZEK G ; BACSI A ; DHARAJIYA N ; SUR S ; HAZRA TK ; BOLDOGH I.** *Mol Immunol.,* 2009, vol. 46 (13), 2505-14
- **OLSEN NK et al.** *Acta Neurol. Scand.,* 1995, vol. 91, 326-329
- **REGENOLD WT et al.** *J. Neurol. Sci.,* 2008, vol. 275, 106-112
- **SEYAMA K et al.** *Acta Neurol. Scand.,* 1989, vol. 80, 561-568
- **DUTTA, R. et al.** *Ann. Neurol.,* 2006, vol. 59, 478-489
- **COPELAND et al.** *Current Biol,* 2009, vol. 19, 1-8
- **LOMB et al.** *Biochim Biophy Acta.,* 2010, vol. 1804 (8), 1652-7
- **BRUSS et al.** *Am. J. Physiol. Edocrinol. Metab.,* 2010, vol. 298, E108-E116
- **LEE et al.** *BMC Bioinformatics,* 2005, vol. 6, 269
- **HUANG DW et al.** *Nature Protoc.,* 2009, vol. 4 (1), 44-57
- **DENNIS G et al.** *Genome Biol.,* 2003, vol. 4 (5), 3
- **JELLALI A. ; MEZIANE H. ; OUAGAZZAL A-M. et al.** *Vis. Res.,* 2005